(19) Europäisches Patentamt
European Patent Office
Office européen des brevets

(11) EP 3 610 866 A1

(12) # EUROPEAN PATENT APPLICATION
published in accordance with Art. 153(4) EPC

(43) Date of publication:
**19.02.2020 Bulletin 2020/08**

(21) Application number: **18784067.3**

(22) Date of filing: **13.04.2018**

(51) Int Cl.:
***A61K 31/194*** *(2006.01)* ***A61K 31/11*** *(2006.01)*
***A61K 31/155*** *(2006.01)*

(86) International application number:
**PCT/KR2018/004344**

(87) International publication number:
**WO 2018/190676 (18.10.2018 Gazette 2018/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.04.2017 KR 20170048558**

(71) Applicant: **National Cancer Center**
**Goyang-si, Gyeonggi-do 10408 (KR)**

(72) Inventor: **KIM, Soo Youl**
**Goyang-si**
**Gyeonggi-do 10382 (KR)**

(74) Representative: **J A Kemp LLP**
**14 South Square**
**Gray's Inn**
**London WC1R 5JJ (GB)**

(54) **PHARMACEUTICAL COMPOSITION FOR PREVENTING AND TREATING CANCER, CONTAINING MALATE-ASPARTATE SHUTTLE INHIBITOR AND ANTICANCER DRUG AS ACTIVE INGREDIENTS**

(57) The present invention relates to a technology for using a malate-aspartate shuttle (MAS) inhibitor as an agent for treating cancer. More particularly, provided is a pharmaceutical composition for preventing or treating cancer, containing, as an active ingredient, phenyl succinic acid, methyl malonic acid, N-(1-pyrenyl)maleimide and phthalonic acid, which are MAS inhibitors, or a mixture of the MAS inhibitor and an anticancer drug.

Figure 3A

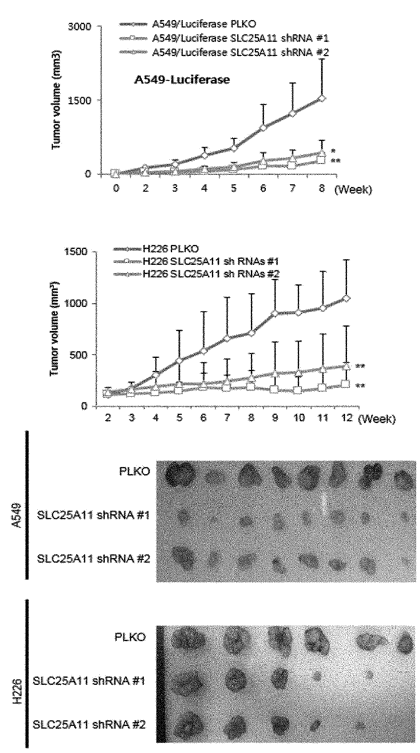


6/21

EP 3 610 866 A1

## Description

[Technical Field]

This application claims priority to and the benefit of Korean Patent Application No. 10-2017-0048558, filed on April 14, 2017, the disclosure of which is incorporated herein by reference in its entirety.

The present invention relates to a technology for using a malate-aspartate shuttle (MAS) inhibitor as a cancer therapeutic agent, and more particularly to a pharmaceutical composition for preventing or treating cancer, which includes, as an active ingredient, an MAS inhibitor or a mixture of the MAS inhibitor and an anticancer agent.

[Background Art]

Cancer refers to a cell mass, which is also referred to as a tumor, consisting of undifferentiated cells that proliferate indefinitely such that necessary conditions in the tissue are ignored, unlike normal cells capable of regularly and controllably proliferating and inhibiting according to the needs of individuals. This unlimited proliferation of cancer cells is an incurable disease that penetrates into surrounding tissues and, more seriously, metastasizes to other organs in the body, causing severe pain and eventually causing death.

Cancer is broadly classified into blood cancer and solid cancer, and it occurs in almost all parts of the body, including pancreatic cancer, breast cancer, oral cancer, liver cancer, uterine cancer, esophageal cancer, skin cancer, and the like. For the treatment of these cancers, a few targeting therapeutic agents such as Gleevec® or Herceptin® have recently been used for the treatment of specific cancer, but to date, surgery or anticancer treatment using radiotherapy and chemotherapy which inhibits cell proliferation is a main treatment method. However, since they are not targeting agents, treatment eventually fails despite the initial successful responses induced by anticancer drugs, mainly due to side effects caused by cytotoxicity and drug resistance, which are the biggest problems of existing chemotherapeutic agents. Therefore, to overcome the limitations of these chemotherapeutic agents, there is a need to continuously develop a targeting agent having an accurate anticancer mechanism.

The pathways for synthesizing ATP are different between normal cells and cancer cells. In normal cells, when glucose is absorbed, sugars degraded by glycosylation produce NADH through the TCA cycle inside the mitochondria, and ATP is produced using the NADH at a mitochondrial membrane potential, thus enabling the cells to use ATP. In contrast, in cancer cells, since glycosylation does not occur, lactic acid is produced by LDH and released to the outside of a cell, and ALDH is overexpressed instead to participate in ATP production. Thus, for the killing of only cancer cells, drugs intended for inducing ATP deficiency in cells by inhibiting the expression or activity of ALDH and thus blocking the proliferation of cells to induce apoptosis are being developed.

Gossypol and phenfonnin are known as anticancer compounds using such intracellular pathways (Korean Registration Publication No. 10-1579371 and Korean Patent Registration No. 10-145806).

Gossypol is a naturally occurring double biphenolic compound derived from *Gossypium* sp. It is known as an inhibitor of aldehyde dehydrogenase (ALDH) *in vivo,* and thus research into the use thereof for treatment has been conducted. According to *in vivo* experiments using gossypol as a male contraceptive, the safety of long-term administration of such a compound has been reported.

Phenformin is a drug belonging to the biguanide family including metformin, and is known as a diabetes treatment agent. However, as biguanide drugs such as phenformin became known to be effective in the treatment of cancers lacking the p53 gene by activating AMP-activated protein kinase (AMPK), which is a key enzyme that physiologically regulates carbohydrate metabolism and lipid metabolism, research into anticancer effects of the phenformin drug was conducted, and it was verified that phenformin induces ATP deficiency by blocking the pathway of converting NADH into ATP by reducing the mitochondrial membrane potential, thereby exhibiting an anticancer effect.

Meanwhile, a structure involved in the transfer passage of a material from the outside to the inside of the mitochondrial inner membrane is referred to as a malate-aspartate shuttle (MAS). The MAS is involved in the movement of malate and aspartate through the pathway proteins MAT and GAT expressed in the mitochondrial inner membrane, and serves to help the movement of NADH. It is known that MAS occurs in various tumor cells, and it is known that MAS is capable of being involved in mitochondrial NADH oxidation-reduction in tumor cell lines, and thus NADH oxidation is reduced through glycosylation in the cytoplasm of cancer cells and NADH oxidation is exhibited in the mitochondria via the MAS (Greenhouse, Walter VV, and Albert L. Lehninger, Cancer Research 36.4 (1976): 1392-1396).

Therefore, the inventors of the present invention anticipated that when the entry of NADH into the mitochondria was blocked by blocking the MAS, ATP deficiency in cancer cells could be more significantly induced via treatment with gossypol or phenformin, and confirmed that cell apoptosis was increased in lung cancer and melanoma cell lines upon co-treatment with an MAS inhibitor and gossypol or phenformin, thus completing the present invention.

[Disclosure]

[Technical Problem]

Therefore, the inventors of the present invention confirmed that, upon administration of a malate-aspartate shuttle (MAS) inhibitor, the MAS inhibitor was capable of exhibiting an effect of inhibiting intracellular ATP production and inhibiting cell proliferation. It was also verified that, upon treatment with a mixture of the MAS inhibitor and gossypol or phenformin, a synergistic effect could be obtained in inhibiting the proliferation of cancer cells and inducing apoptosis thereof, and thus completed the present invention.

Therefore, an object of the present invention is to provide a pharmaceutical composition for preventing or treating cancer.

[Technical Solution]

To achieve the above object, the present invention provides a pharmaceutical composition for preventing or treating cancer, which includes a malate-aspartate shuttle (MAS) inhibitor as an active ingredient.

The present invention also provides a pharmaceutical composition for preventing or treating cancer, which includes the above-described pharmaceutical composition and an anticancer agent.

The present invention also provides a method of treating cancer, the method including administering an effective amount of a malate-aspartate shuttle inhibitor to an individual in need thereof.

The present invention also provides a method of treating cancer, the method including administering effective amounts of an MAS inhibitor and an anticancer agent to an individual in need thereof.

The present invention also provides a use of a pharmaceutical composition for preventing or treating cancer, the pharmaceutical composition including a malate-aspartate shuttle inhibitor.

The present invention also provides a use of a pharmaceutical composition for preventing or treating cancer, the pharmaceutical composition including a malate-aspartate shuttle inhibitor and an anticancer agent.

In an exemplary embodiment of the present invention, the MAS inhibitor may inhibit the expression or activity of a protein included in MAS. In this regard, the protein included in MAS may be any one or more selected from the group consisting of malate-$\alpha$-ketoglutarate transporter (MAT), glutamate-aspartate transporter (GAT), malate dehydrogenase 1, malate dehydrogenase 2, glutamic oxaloacetic transaminase 1, and glutamic oxaloacetic transaminase 2. In addition, the siRNA may be any one of a forward siRNA having a nucleotide sequence represented by SEQ ID NO: 1 and a reverse siRNA having a nucleotide sequence represented by SEQ ID NO: 2; and a forward siRNA having a nucleotide sequence represented by SEQ ID NO: 3 and a reverse siRNA having a nucleotide sequence represented by SEQ ID NO: 4, and the shRNA may be any one of a nucleotide sequence represented by SEQ ID NO: 5 and a nucleotide sequence represented by SEQ ID NO: 6.

In addition, the MAS inhibitor may be any one or more selected from the group consisting of phenyl succinic acid, methyl malonic acid, N-(1-pyrenyl)maleimide, phthalonic acid, methyl 3-(3-(4-(2,4,4-trimethylpentan-2-yl)phenoxy)-propanamido)benzoate), LW6, 2-thenoyl-trifluoroacetone, chlorothricin, aminooxyacetic acid (AOA), hydrazinosuccinate, 2-amino-3-butenoic acid, and a mercurial reagent.

In an exemplary embodiment of the present invention, the cancer may be any one or more selected from the group consisting of lung cancer, melanoma, uterine cancer, breast cancer, gastric cancer, brain cancer, rectal cancer, colon cancer, skin cancer, blood cancer, liver cancer, ovarian cancer, kidney cancer, prostate cancer, and pancreatic cancer.

In an exemplary embodiment of the present invention, the anticancer agent may be any one or more selected from the group consisting of gossypol, phenformin, and etomoxir, and the anticancer agent and the MAS inhibitor may be mixed in a molar ratio of 1:10 to 500.

[Advantageous Effects]

Therefore, the present invention provides a pharmaceutical composition for preventing or treating cancer, which includes, as an active ingredient, a malate-aspartate shuttle (MAS) inhibitor; or a drug mixture of the MAS inhibitor and an anticancer agent.

The MAS inhibitor of the present invention may inhibit the growth of cancer cells by inhibiting intracellular ATP production. In addition, in the case in which cancer cells are treated with the MAS inhibitor along with an anticancer agent such as gossypol or phenformin, since intracellular ATP deficiency is induced specifically to the cancer cells, the MAS inhibitor and the anticancer agent may synergistically work to not only inhibit tumor growth by inhibiting cell proliferation but also exhibit a significant cancer cell killing effect, thus being effective in killing cancer which has occurred. In this regard, while significant cell killing does not occur in normal cells, the MAS inhibitor and the anticancer agent

exhibit a synergistic effect on cancer cells, and thus an enhanced cancer treatment effect can be obtained compared to the case of treatment with the MAS inhibitor or the anticancer agent alone, and accordingly, the composition of the present invention can be effectively used for cancer treatment.

[Description of Drawings]

FIG. 1 illustrates the effect of inhibiting cell proliferation in cancer cell lines when the expression of a protein contained in the malate-aspartate shuttle (MAS) was inhibited, wherein:

FIG. 1A illustrates the results of confirming a cell proliferation inhibitory effect according to the inhibition of expression of the SLC25A11 protein, which is a protein contained in the MAS in lung cancer cell lines and melanoma cell lines;
FIG. 1B illustrates the results of confirming cell apoptosis according to the inhibition of SLC25A11 protein expression in lung cancer cell lines and melanoma cell lines; and
FIG. 1C illustrates the results of confirming a decrease in expression level of the SLC25A11 protein through SLC25A11 shRNA in lung cancer cell lines and melanoma cell lines.

FIG. 2 illustrates the effect of inhibiting intracellular ATP production when the MAS was inhibited in cancer cell lines, wherein:

FIG. 2A illustrates the results of confirming a decrease in ATP expression level according to the inhibition of SLC25A11 expression in cancer cell lines; and
FIG. 2B illustrates the results of confirming changes in expression levels of intracellular metabolism-related proteins in cancer cell lines according to the inhibition of SLC25A11 expression.

FIG. 3 illustrates the effect of inhibiting tumor growth according to MAS inhibition in a lung cancer animal model, wherein:

FIG. 3A illustrates the results of confirming changes in tumor volume of mice, into which lung cancer cell lines were transplanted, according to the inhibition of SLC25A11 expression; and
FIG. 3B illustrates the results of comparing tumor weights of mice, into which lung cancer cell lines were transplanted, according to the inhibition of SLC25A11 expression.

FIG. 4 illustrates the results of confirming the effect of inhibiting cancer cell growth according to treatment with an MAS activity inhibitor, wherein:

FIG. 4A is a graph confirming the effect of inhibiting cancer cell growth when a lung cancer cell line was treated with phenyl succinic acid (PSA);
FIG. 4B is a graph confirming the effect of inhibiting cancer cell growth when a melanoma cell line was treated with PSA; and
FIG. 4C is a graph comparing the proliferation rates of cells of a normal control treated with PSA.

FIG. 5 illustrates the results of confirming the effect of inhibiting intracellular ATP production according to treatment with an MAS inhibitor.
FIG. 6 illustrates a synergistic effect on cancer cell apoptosis through co-treatment with an MAS inhibitor and gossypol, wherein:

FIG. 6A illustrates the results of confirming the effect of inhibiting cell proliferation and inducing cell apoptosis when cancer cell lines were co-treated with PSA or PA, which is an MAS inhibitor, and gossypol;
FIG. 6B illustrates the results of confirming a synergistic effect on intracellular ATP deficiency when cancer cell lines were co-treated with an MAS inhibitor and gossypol; and
FIG. 6C illustrates the results of confirming the effect of inhibiting cell proliferation and inducing cell apoptosis when cancer cell lines were co-treated with NPM, which is an MAS inhibitor, and gossypol.

FIGS. 7A and 7B illustrate the results of confirming a synergistic effect on decreasing a mitochondrial membrane potential through co-treatment with an MAS inhibitor and gossypol.

FIGS. 8A and 8B illustrate the results of confirming cell apoptosis due to co-treatment with an MAS inhibitor and gossypol.

FIGS. 9A, 9B, and 9C illustrate the results of confirming a synergistic effect on cancer cell proliferation inhibition through co-administration of an MAS inhibitor and phenformin.

FIG. 10 illustrates the results of confirming a synergistic effect on cancer cell proliferation inhibition through co-administration of an MAS inhibitor and etomoxir.

FIG. 11 is a schematic view illustrating a malate-aspartate shuttle pathway.

[Best Mode]

Hereinafter, the present invention will be described in detail.

As described above, the inventors of the present invention anticipated that when the entry of NADH into the mitochondria was blocked by blocking the malate-aspartate shuttle (MAS), ATP deficiency in cancer cells could be more significantly induced through treatment with gossypol or phenformin.

The MAS inhibitor of the present invention may inhibit the growth of cancer cells by inhibiting intracellular ATP production. In addition, in the case in which cancer cells are treated with the MAS inhibitor together with an anticancer agent such as gossypol or phenformin, since intracellular ATP deficiency is induced specifically to the cancer cells, the MAS inhibitor and the anticancer agent may exhibit a synergistic effect on cell proliferation inhibition, and accordingly, not only inhibit tumor growth by inhibiting cell proliferation, but also exhibit a significant cancer cell apoptosis effect, thus being effective in killing cancer which has occurred.

Therefore, the present invention provides a pharmaceutical composition for preventing or treating cancer, which includes a malate-aspartate shuttle (MAS) inhibitor as an active ingredient.

The present invention also provides a pharmaceutical composition for preventing or treating cancer, which includes the MAS inhibitor and an anticancer agent.

In the pharmaceutical composition of the present invention, the MAS inhibitor may be an inhibitor against the expression or activity of a protein contained in MAS.

In the present invention, the term "protein contained in MAS" refers to a protein known in the art as a component of the malate-aspartate shuttle. Specifically, as illustrated in FIG. 11, a total of 6 proteins are contained in the malate-aspartate shuttle, and any MAS inhibitor may be included without limitation as long as it can be understood by those of ordinary skill in the art as being capable of inhibiting the activity of one or more proteins selected from the six proteins. More specifically, the protein contained in the MAS may be any one or more selected from the group consisting of malate-$\alpha$-ketoglutarate transporter (MAT), glutamate-aspartate transporter (GAT), malate dehydrogenase 1 (MDH1), malate dehydrogenase 2 (MDH2), glutamic oxaloacetic transaminase 1 (GOT1), and glutamic oxaloacetic transaminase 2 (GOT2), but the present invention is not limited thereto. The MAT is a transporter protein encoded by the human SLC25A11 gene and may also be referred to as a mitochondrial 2-oxoglutarate/malate carrier protein. The GAT is a transporter protein encoded by the human SLC25A12 gene and may also be referred to as a calcium-binding mitochondrial carrier protein Aralarl. Among the malic acid dehydrogenases, MDH1 is present in the cytoplasm (cytosolic form), and MDH2 is present in the mitochondria (mitochondrial form). In addition, among the glutamic oxalacetic transaminases, GOT1 is present in the cytoplasm and GOT2 is present inside the mitochondria. The GOT1 and the GOT2 may also be referred to as aspartate aminotransferase 1 (AST1) and AST2, respectively.

The "inhibitor against the expression or activity of a protein contained in MAS" according to the present invention may be particularly a compound that inhibits the expression of a protein contained in MAS using siRNA or shRNA or inhibits the activity of the MAS protein.

In the "inhibition of the expression of a protein contained in MAS using siRNA or shRNA", the siRNA may be any one of a forward siRNA having a nucleotide sequence represented by SEQ ID NO: 1 and a reverse siRNA having a nucleotide sequence represented by SEQ ID NO: 2; and a forward siRNA having a nucleotide sequence represented by SEQ ID NO: 3 and a reverse siRNA having a nucleotide sequence represented by SEQ ID NO: 4, the shRNA may be any one or more selected from a nucleotide sequence represented by SEQ ID NO: 5 and a nucleotide sequence represented by SEQ ID NO: 6, but the present invention is not limited thereto. That is, any siRNA or shRNA which may be selected by those of ordinary skill in the art for inhibiting the expression of a protein contained in MAS may be used without limitation.

The "compound that inhibits the activity of the MAS protein" may be any one or more selected from the group consisting of phenyl succinic acid, methyl malonic acid, N-(1-pyrenyl)maleimide)phthalonic acid, methyl 3-(3-(4-(2,4,4-trimethylpentan-2-yl)phenoxy)- propanamido)benzoate), LW6, 2-thenoyl-trifluoroacetone, chlorothricin, aminooxyacetic acid (AOA), hydrazinosuccinate, 2-amino-3-butenoic acid, and a mercurial reagent, but the present invention is not limited thereto. That is, any material which may be selected by those of ordinary skill in the art for inhibiting the expression or activity of a protein contained in MAS may be used without limitation. Specifically, among the above-listed compounds that inhibit the activity of the MAS protein, methyl 3-(3-(4-(2,4,4-trimethylpentan-2-yl)phenoxy)-propanamido)benzoate),

LW6, 2-thenoyl-trifluoroacetone, and chlorothricin are known as an inhibitor against the MDH enzyme (Naik, Ravi, et al. Journal of medicinal chemistry 60.20 (2017): 8631-8646; Eleftheriadis, Theodoros, et al. Experimental and therapeutic medicine 10.5 (2015): 1959-1966; Gutman, Menachem, and Ester Hartstein. FEBS letters 49.2 (1974): 170-173.; SCHINDLER, Peter W. The FEBS Journal 51.2 (1975): 579-585.). In addition, AOA, hydrazinosuccinate, and 2-amino-3-butenoic acid are known as an inhibitor against the GOT enzyme (Wang, Caixia, et al. Cancer letters 378.1 (2016): 1-7.; Yamada, Ryo-Hei, et al. Biochimica et BiophysicaActa (BBA)-General Subjects 801.1 (1984): 151-154.; Rando, Robert R. Biochemistry 13.19 (1974): 3859-3863.). In addition, maleimide-based compounds, including N-(1-pyrenyl)maleimide and mercurial-based compounds are known as SLC25A11 inhibitors (Capobianco, Loredana, et al. Biochemistry 35.27 (1996): 8974-8980.).

In the pharmaceutical composition of the present invention, the "cancer" may be any one or more selected from the group consisting of lung cancer, melanoma, uterine cancer, breast cancer, gastric cancer, brain cancer, rectal cancer, colon cancer, skin cancer, blood cancer, liver cancer, ovarian cancer, kidney cancer, prostate cancer, and pancreatic cancer, but the present invention is not limited thereto.

In the pharmaceutical composition of the present invention, the "anticancer agent" may be an anticancer agent which is capable of regulating a mitochondrial membrane potential in cancer cells or, when it is an anticancer agent capable of inducing ATP deficiency in the cancer cells and is used together with the MAS inhibitor, is capable of exhibiting a synergistic effect on cancer treatment. Specifically, the anticancer agent may be any one or more selected from the group consisting of gossypol, phenformin, and etomoxir.

The "gossypol" acts as an inhibitor against the intracellular expression and activity of ALDH. Specifically, in a cellular mechanism wherein ALDH produces NDAH in the intracellular serine-folate mechanism and ATP is generated therefrom, gossypol may act as an inhibitor against ALDH expression or activity and thereby induce intracellular ATP deficiency, resulting in cancer cell apoptosis. The gossypol has a structure represented by Formula 1 below:

[Formula 1]

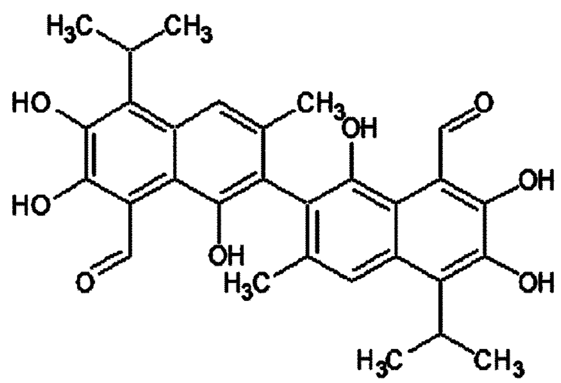

The "phenformin" of the composition according to the present invention acts as an inhibitor against mitochondria complex I in a cell. Specifically, phenformin may reduce a mitochondrial membrane potential through inhibition of the activity of mitochondria complex I, which leads to reduction in intracellular ATP synthesis, and thus cancer cells may be effectively killed. The phenformin has a structure represented by Formula 2 below:

[Formula 2]

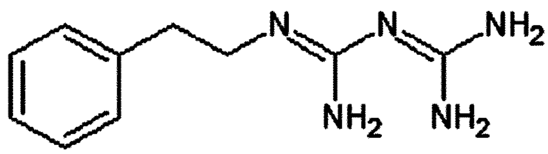

"Etomoxir" of the composition according to the present invention serves to inhibit intracellular b-oxidation. Specifically, etomoxir may irreversibly inhibit the activity of carnitine palmitoyltransferase-1 (CPT-1) located outside the inner mitochondrial membrane, thereby blocking the transfer of a fatty acid acyl ring from the cytoplasm into the mitochondrial membrane, and thus serves to inhibit ATP production caused by fatty acid oxidation. The etomoxir has a

structure represented by Formula 3 below:

[Formula 3]

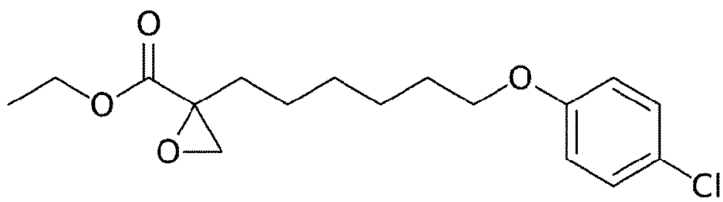

In the pharmaceutical composition of the present invention, it is more preferable that "the MAS inhibitor" is provided in a mixed form for co-treatment with an anticancer agent. In mixing, it is preferable that the MAS inhibitor and the anticancer agent are mixed in a molar ratio of 10: 1 to 500:1. In particular, it is more preferable that the MAS inhibitor and the anticancer agent are mixed in a molar ratio of 30:1 to 450: 1. More particularly, it is most preferable that the MAS inhibitor and the anticancer agent are mixed in a molar ratio of 40:1 to 400:1, but the present invention is not limited thereto.

More specifically, the pharmaceutical composition of the present invention may include the MAS inhibitor at a concentration of 0.1 mM to 10 mM. In this regard, since the anticancer agent may be mixed with the MAS inhibitor in the above-described mixing ratio, the anticancer agent may be used at a concentration ranging from 0.2 μM to 1 mM, preferably 1 μM to 500 μM, and more preferably 10 μM to 100 μM, which can be selected by those of ordinary skill in the art.

In specific embodiments of the present invention, first, the inventors of the present invention examined whether a cancer treatment effect could be obtained by the inhibition of MAS expression. In the present invention, GAT was selected as a protein contained in MAS, and to inhibit the expression of the MAS protein, i.e., SLC25A11, which is a GAT subtype, siRNA or shRNA was introduced into lung cancer cell lines and melanoma cell lines. As a result, it was confirmed that, when the expression of SLC25A11, which is an MAS protein, was inhibited, intracellular ATP production was reduced and the cell proliferation rate was significantly reduced, as compared to a normal control (see FIGS. 1 and 2). In addition, as a result of examining the degree of tumor growth in cancer-cell-line xenograft mice to confirm whether tumor growth can be inhibited even *in vivo* when the expression of the MAS protein is inhibited, the inventors of the present invention confirmed that the degree of tumor growth was insignificant in mice into which cancer cells with suppressed MAS protein expression had been transplanted (see FIG. 3).

To confirm whether the same cancer treatment effect can be exhibited even in the case in which not only the expression of the MAS protein but also the activity thereof are blocked, the effect of inhibiting cancer cell growth according to treatment with an MAS activity inhibitor was examined. As a result of culturing a cancer cell line after phenyl succinic acid (PSA) was added as an MAS activity inhibitor to the medium, it was confirmed that, when the activity of the MAS protein was inhibited, intracellular ATP production was reduced (see FIG. 5), and cell proliferation was also inhibited (see FIG. 4).

In addition, the inventors of the present invention had confirmed that the MAS inhibitor was capable of inhibiting cancer cell proliferation through the inhibition of ATP production in the inner mitochondrial membrane of a cancer cell, and thus examined whether a significant cancer treatment effect is obtained by the co-treatment with the MAS inhibitor and an anticancer drug capable of regulating a mitochondrial membrane potential and intracellular ATP production. As a result, it was confirmed that, when PSA or phthalonic acid (PA), which is an MAS inhibitor, was mixed with gossypol, which is an anticancer agent, and used for co-treatment, an increased synergistic effect on reducing a mitochondrial membrane potential could be exhibited compared to the case of treatment with the MAS inhibitor or the anticancer agent alone (see FIG. 7). In addition, not only the inhibition of cancer cell proliferation but also an increased cell apoptosis effect were confirmed, through which it was confirmed that PSA or PA and gossypol were able to not only inhibit tumor growth, but also exhibit a tumor apoptosis effect (see FIGS. 6 and 8). It was also confirmed that in the case of co-treatment with an MAS inhibitor and phenformin having been mixed, a significant synergistic effect on cancer cell proliferation inhibition could be exhibited (see FIG. 9). In contrast, it was confirmed that when normal cells were treated with a mixture of the MAS inhibitor and the anticancer agent, cell proliferation inhibition and cell apoptosis were not induced, from which it was confirmed that the effect of specifically killing only cancer cells could be exhibited by treatment with a mixture of the MAS inhibitor of the present invention and an anticancer agent.

Thus, the MAS inhibitor of the present invention may inhibit cancer cell growth by inhibiting intracellular ATP production. In addition, in the case in which cancer cells are treated with the MAS inhibitor together with an anticancer agent such as gossypol or phenformin, intracellular ATP deficiency is induced specifically to the cancer cells, and thus the MAS inhibitor and the anticancer agent exhibit a synergistic effect on inhibiting cell proliferation, and therefore, not

only inhibit tumor growth by inhibiting cell proliferation, but also exhibit a significant cancer cell apoptosis effect, thus being effective in killing cancer which has occurred. In this regard, while significant cell killing does not occur in normal cells, the MAS inhibitor and the anticancer agent exhibit a synergistic effect on cancer cells, and thus an enhanced cancer treatment effect can be obtained compared to the case of treatment with the MAS inhibitor or the anticancer agent alone, and accordingly, the composition of the present invention can be effectively used for cancer treatment.

In addition, the pharmaceutical composition for preventing or treating cancer of the present invention may further include an anticancer agent. In this regard, a suitable anticancer agent may be any one or more selected from the group consisting of nitrogen mustard, imatinib, oxaliplatin, rituximab, erlotinib, netatinib, lapatinib, zefitinib, vandetanib, nirotinib, semasanib, bosutinib, axitinib, cediranib, lestaurtinib, trastuzumab, gefitinib, bortezomib, sunitinib, carboplatin, sorafenib, bevacizumab, cisplatin, cetuximab, viscumalbum, asparaginase, tretinoin, hydroxycarbamide, dasatinib, estramustine, gemtuxumabozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacytidine, methotrexate, uracil, cytarabine, fluorouracil, fludarabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, tretoin, exemestane, aminogluthetimide, anagrelide, navelbine, fadrozole, tamoxifen, toremifene, testolactone, anastrozole, letrozole, vorozole, bicalutamide, lomustine, and carmustine, but the present invention is not limited thereto. More preferably, the anticancer agent may have ALDH inhibitory activity as in gossypol or may be a biguanide drug such as phenformin.

When the composition of the present invention is used as a medicine, a pharmaceutical composition including gossypol and phenformin may be formulated and administered in various oral or parenteral dosage forms as described below upon clinical administration, but the present invention is not limited thereto.

Preparations for oral administration include, for example, tablets, pills, hard/soft capsules, liquids, suspensions, emulsions, syrups, granules, elixirs, and the like. These preparations include, in addition to the active ingredient, a diluent (e.g., lactose, dextrose, sucrose, mannitol, sorbitol, cellulose, and/or glycine), and a lubricant (e.g., silica, talc, stearic acid and magnesium or calcium salts thereof, and/or polyethylene glycol). Tablets may also include a binder such as magnesium aluminum silicate, starch paste, gelatin, methyl cellulose, sodium carboxymethylcellulose, and/or polyvinyl pyrrolidone, and in some cases, may include a disintegrating agent such as starch, agar, alginic acid or sodium salts thereof, or boiling mixture and/or an absorbent, a coloring agent, a flavoring agent, and a sweetening agent.

The pharmaceutical composition of the present invention, which includes the MAS inhibitor and an anticancer agent, may be administered parenterally, and parenteral administration is performed via subcutaneous injection, intravenous injection, intramuscular injection, or intrathoracic injection. In this regard, to formulate preparations for parenteral administration, gossypol and phenformin are mixed with a stabilizer or a buffer in water to prepare a solution or a suspension, followed by preparation thereof into an ampoule or vial unit dosage form. The composition may be sterilized and/or include an adjuvant such as a preservative, a stabilizer, wettable powder, an emulsion promoter, a salt for the control of osmotic pressure, and/or a buffer, and other therapeutically effective materials, and may be formulated using a conventional method, such as mixing, granulation, or coating.

In addition, in the present invention, a dose of the MAS inhibitor or a drug mixture of the MAS inhibitor and gossypol and/or phenformin, which is to be administered to the human body, may vary depending on the age, body weight, and gender of patients, administration forms, health conditions, and the severity of diseases, may generally range from 0.001 mg/day to 1,000 mg/day, preferably 0.01 mg/day to 500 mg/day, with respect to an adult patient with a body weight of 60 kg, and may also be administered once a day or in multiple doses at regular intervals in accordance with the prescription of a doctor or a pharmacist.

In addition, in the pharmaceutical composition of the present invention, which includes the MAS inhibitor or a drug mixture of gossypol and/or phenformin, the MAS inhibitor, the gossypol, and the phenformin may be prepared into a preparation for oral administration, which includes a pharmaceutically acceptable salt, hydrate, or solvate thereof.

The preparation for oral administration of the present invention may be a sustained-release preparation or a controlled-release preparation. In the case of the sustained-release preparation, the MAS inhibitor and the anticancer agent may be simultaneously released, and in the case of the controlled-release preparation, the release may be controlled such that the MAS inhibitor and the anticancer agent, or the anticancer agent and the MAS inhibitor are sequentially released.

[Mode of the Invention]

Hereinafter, the present invention will be described in further detail with reference to the following examples. These examples are provided for illustrative purposes only, and it will be obvious to those of ordinary skill in the art that

these examples are not construed as limiting the scope of the present invention.

## [Example 1] Confirmation of Cancer Cell Growth Inhibitory Effect according to MAS Inhibition

### <1-1> Confirmation of Lung Cancer and Melanoma Cell Growth Inhibitory Effect by MAS Inhibition

It was examined whether the effect of inhibiting cancer cell proliferation could be obtained when the expression of SLC25A11, which is a subtype of GAT, a protein contained in the malate-aspartate shuttle, was inhibited.

Specifically, A549 cells, H522 cells, H226 cells, H23 cells, and EKVX cells, which are lung cancer cell lines; and UACC62 cells, UACC257 cells, A375 cells, SK-MEL-5 cells, and MALME-3M cells, which are melanoma cell lines, were separately cultured. The respective cells were cultured at a 100 ml dose and inoculated in a 96-well plate at a concentration ranging from 5,000 cells/ml to 20,000 cells/ml according to the doubling time of each cell line. Then, 20 μM 2SLC25A11 siRNA was added to the plate, which had been inoculated with cells, and incubated in a $CO_2$ incubator for 2 weeks. After completion of the culturing, to perform SRB analysis, a cooled 50% aqueous TCA solution was added to each well to a final concentration of 10% and the cells were incubated in a 4 □ refrigerated state for 60 minutes and then fixed. After incubation, the supernatant was removed, washed five times with tap water, and dried. A 1% acetic acid aqueous solution containing 0.4% sulforhodamine B was added to the dried sample in each well and maintained at room temperature for 10 minutes to stain the cells. After staining, the dye which was not used for staining was removed by washing with a 1% acetic acid solution, and then the plate was dried again. The dye which was used for staining was dissolved in a 10 mM Trisma base solution, and then absorbance was measured at 515 nm.

In addition to the transduction of siRNA, the inhibition of SLC25A11 expression was induced by expressing shRNA in the cells. A shRNA expression vector targeting SLC25A11 was transduced into a target cancer cell line, and then the cells were stained with crystal violet to observe the number of viable cells, and the cells were disrupted and subjected to western blotting to compare expression levels.

As a result, as illustrated in FIG. 1, it was confirmed that, when the expression of SLC25A11, which is an MAS protein, was inhibited in lung cancer and melanoma cell lines, the expression of the SLC25A11 protein was significantly reduced compared to a normal control (see FIG. 1C), and accordingly, the number of viable cells was reduced and cell proliferation rates were significantly reduced (see FIGS. 1A and 1B).

### <1-2> Confirmation of ATP Production Inhibitory Effect according to MAS Inhibition

Since it had been confirmed that cell growth was inhibited when the expression of the MAS protein was inhibited in cancer cell lines, intracellular ATP production levels were examined.

Specifically, A549 cells, IMR90 cells, H23 cells, H226 cells, H522 cells, and EKVX cells, which are lung cancer cell lines; and UACC62 cells, MALME-3M cells, A357 cells, M14 cells, and UACC257 cells, which are melanoma cell lines, were separately cultured. 40 nM SLC25A11 siRNA was added to the cultured cells and incubated in a $CO_2$ incubator for 48 hours, and then intracellular ATP levels were examined using an ATP colorimetric/fluorometric analysis kit (Bio-Vision, Milpitas, CA, USA) in accordance with the manufacturer's protocol. The incubated cells were divided into groups of 1 x $10^6$ cells, 100 μl of an ATP assay buffer was added thereto to lyse the cells, followed by centrifugation at 15,000 xg and 4 □ for 2 minutes to separate only the supernatant. 2 μl to 50 μl of the separated supernatant was transferred to a 96-well plate, and then an ATP assay buffer was added thereto to a final volume of 50 μl per well. Thereafter, 50 μl of an ATP reaction mixture containing 44 μl of an ATP assay buffer, 2 μl of an ATP probe, 2 μl of an ATP converter, and 2 μl of a developer mixture was added to each well of the 96-well plate and mixed. After mixing, the plate was maintained in a dark room at room temperature for 30 minutes, and then absorbance was measured at 570 nm using a microplate reader. To compare relative ATP levels among the measured values, an absorbance value for cancer cells to which gossypol and phenformin were not added was set as a reference, and relative absorbance values of the cases in which cancer cells were treated with each drug were compared, to compare ATP levels in the cells.

For UACC62 cells, expression levels of metabolites of various metabolic pathways according to not only ATP inhibition but also the inhibition of MAS protein expression were also compared. 40 nM SLC25A11 siRNA was added to UACC62 cells and incubated in a $CO_2$ incubator for 24 hours, and then expression levels of metabolites of the TCA cycle metabolism pathway and the pentose phosphate metabolism pathway were examined by LC-MS/MS.

As a result, as illustrated in FIG. 2, it was confirmed that intracellular ATP levels were reduced in cancer cell lines in which the MAS was inhibited by treatment with SLC25A11 siRNA (see FIG. 2A), and that the expression levels of metabolites of the metabolism pathways were also significantly reduced (see FIG. 2B).

**[Example 2] Confirmation of Tumor Growth Inhibitory Effect according to MAS Inhibition in Lung Cancer Animal Model**

Since it had been confirmed that, at a cellular level, the effect of inhibiting cancer cell growth could be exhibited through the inhibition of an expression level of the MAS protein, it was then examined whether the effect of inhibiting tumor growth could be exhibited even *in vivo.*

First, 6-week-old to 8-week-old Balb/c-nu mice (Central Lab. Animal, Highland Heights, KY, USA) were prepared to construct a cancer mouse model. In addition, Luciferase SLC25A11 shRNA was introduced into A549 cells or H226 cells and cultured, and for each case, 5.0 x $10^6$ cells were subcutaneously injected into the prepared mice using a 1 ml syringe. The mice were raised for 2 weeks to examine the tumor size of each mouse. Initial tumor sizes after cancer cell injection were measured using a caliper. Tumor volume was obtained using Equation 1 below:

$$\text{Volumn}(mm^3) = \frac{\text{long diameter X short diameter}^2}{2}$$

[Equation 1]

As a result, as illustrated in FIG. 3, it was confirmed that the level of increase in tumor volume was also low in a mouse model in which SLC25A11 shRNA had been introduced into tumors formed by transplantation of a lung cancer cell line, compared to a control mouse model in which SLC25A11 shRNA had been introduced (see FIG. 3A). After the raising was completed, the mice were sacrificed to compare tumor sizes and weights, and even in this case, it was confirmed that the levels of increase in tumor weight and tumor size were significantly reduced in the SLC25A11 shRNA-introduced model group compared to a control (PLKO) (see FIG. 3B).

**[Example 3] Confirmation of Cancer Cell Growth Inhibitory Effect according to Treatment with MAS Activity Inhibitor**

**<3-1> Confirmation of Cancer Cell Growth Inhibitory Effect according to Treatment with Phenyl Succinic Acid (PSA)**

Although it had been confirmed that, both *in vitro* and *in vivo,* tumor growth could be inhibited when MAS expression was suppressed using siRNA or shRNA, it had also been confirmed that it was not possible to completely inhibit cancer cell line growth and tumor growth. Thus, it was examined whether cancer could be effectively inhibited by treatment with an MAS inhibitor.

Specifically, A549 cells (lung cancer cell line), UACC62 cells (melanoma cell line), and IMR90 cells (normal control, lung fibroblasts) were separately cultured. Phenyl succinic acid (PSA), which is an SLC25A11 inhibitor, was added to a culture medium of respective cells at a concentration of 2 mM, 4 mM, 6 mM, 8 mM, or 10 mM, and the cells were cultured at a 100 ml dose for 48 hours, and then cell proliferation levels were examined in the same manner as in Example <1-1> described above, through SRB analysis.

As a result, as illustrated in FIG. 4, it was confirmed that, while the level of cancer cell line proliferation was reduced in a manner dependent upon the concentration of added PSA when lung cancer and melanoma cell lines were treated with PSA, which is an inhibitor against the MAS protein, i.e., SLC25A11 (see FIGS. 4A and 4B), cancer cell proliferation was not significantly inhibited in IMR90, which is a normal cell line, even upon treatment with PSA (see FIG. 4C).

**<3-2> Confirmation of ATP Production Inhibitory Effect according to Treatment with MAS Inhibitor**

Since it had been confirmed that cancer cell line proliferation could be significantly inhibited upon treatment with an MAS inhibitor, it was examined whether ATP levels were reduced in cancer cells upon treatment with an MAS inhibitor.

Specifically, A549 cells (lung cancer cell line) and UACC62 cells (melanoma cell line) were separately cultured. PSA or phthalonic acid (PA) was added to the cultured cells at a concentration of 2 mM, 4 mM, 6 mM, 8 mM, or 10 mM and incubated for 48 hours, and then intracellular ATP levels were examined in the same manner as in Example <1-2> described above.

As a result, as illustrated in FIG. 5, it was confirmed that intracellular ATP levels were reduced in cancer cell lines in which MAS activity was inhibited by treatment with PSA or PA, compared to an untreated control (see FIG. 5).

## [Example 4] Confirmation of Synergistic Effect on Cancer Cell Proliferation Inhibition through Co-treatment with MAS inhibitor and Gossypol

### <4-1> Confirmation of Synergistic Effect on Cancer Cell Apoptosis via Co-treatment with MAS inhibitor and Gossypol

Although it had been confirmed that cancer cell proliferation could be inhibited when cancer cells were treated with the MAS inhibitor, it had also been confirmed that a complete anticancer effect was not exhibited upon treatment with PSA or PA at a concentration of 2 mM to 10 mM. Therefore, the inventors of the present invention examined whether cancer cell proliferation could be more effectively inhibited by co-treating gossypol, which is an anticancer agent capable of inhibiting cancer cell proliferation through the inhibition of ATP production in cancer cells, with the MAS inhibitor.

Specifically, EKVX cells, A549 cells, and HOP-62 cells, which are lung cancer cell lines; UACC62 cells, UACC257 cells, and A375 cells, which are melanoma cell lines; and IMR90 cells, which are lung fibroblasts and a normal control, were separately cultured. 4 mM PSA, 4 mM PA, or 25 $\mu$M N-(1-pyrenyl)maleimide (NPM) was mixed with 10 $\mu$M gossypol, and the resulting mixture was added to a culture medium of each cell line, and further cultured for 48 hours. Then, cell proliferation levels were examined in the same manner as in Example <1-1> described above, through SRB analysis, and intracellular ATP levels were examined in the same manner as in Example <1-2> described above.

As a result, as illustrated in FIG. 6, it was confirmed that while there was no significant difference in intracellular ATP production level between the case of treatment with 10 $\mu$M gossypol alone and the case of treatment with the MAS inhibitor, i.e., PSA or PA alone at a concentration of 4 mM (see FIG. 6B), the reduction in the cell proliferation level was more prominent in the case of treatment with gossypol (see FIG. 6A). It was also confirmed that the effect of inhibiting a cell proliferation level was more significantly exhibited in the case of treatment with gossypol alone than in the case of treatment with NPM, which is an MAS inhibitor, at a concentration of 25 $\mu$M (see FIG. 6C). However, it was confirmed that the effect of inhibiting cell proliferation was more significantly exhibited in an experimental group co-treated with a mixture of the MAS inhibitor and gossypol, and not only cell proliferation was inhibited, but cell apoptosis was also exhibited, resulting in a reduction in the number of cancer cell lines (see FIGS. 6A and 6C). On the other hand, it was confirmed that, while cell proliferation was reduced to a certain extent in the cell line IMR90, which was a normal control, the level of decrease in cell proliferation was insignificant compared to when the same concentrations of the MAS inhibitor and gossypol were used for treatment (see FIG. 6A).

### <4-2> Confirmation of Synergistic Effect on Reduction in Mitochondrial Membrane Potential upon Co-treatment with MAS inhibitor and Gossypol

Since it had been confirmed that cancer cell proliferation was inhibited to a significantly increased extent upon co-treatment with an MAS inhibitor and gossypol compared to the case of treatment with an MAS inhibitor or gossypol alone, and not only the number of cells was reduced, but cancer cell apoptosis to a significant extent was also exhibited, and that intracellular ATP levels were also reduced significantly, it was examined whether there was a change in mitochondrial membrane potential level caused by co-treatment with an MAS inhibitor and gossypol.

Specifically, A549 cells, UACC62 cells, and IMR90 cells, as a normal control, were cultured. The respective cultured cells were treated with a drug mixture of 4 mM of an SLC25A11 inhibitor (PSA or PA), which is an MAS inhibitor, and 10 $\mu$M gossypol or with only one of the drugs and cultured in the same manner as described above for 48 hours, and then each cell culture solution was dispensed into chamber slides (for fluorescence microscopy) or a 6-well plate (for flow cytometry). The dispensed culture solution was treated with 100 nM tetramethylrodamine ester (TMRE) as a fluorescent probe and a reaction was allowed to occur therebetween for 20 minutes. After the reaction, the cells were washed with cooled PBS, and the fluorescence development of the cells was measured using a Zeiss LSM510 fluorescence microscope (Carl Zeiss, Oberkochen, Baden-Wurttemberg, Germany). In addition, fluorescence intensity was analyzed in a flow cytometer using a 585 nm (FL-2) channel.

As a result, as illustrated in FIG. 7, it was confirmed that, while there was no significant change in mitochondrial membrane potential in IMR90 cells (normal control) regardless of treatment with an MAS inhibitor or gossypol alone or co-treatment therewith, there was a change in mitochondrial membrane potential in A549 cells and UACC62 cells. In relation to the above change, it was confirmed that while a significant change in mitochondrial membrane potential was exhibited in A549 cells upon treatment with PSA or PA alone, which is an MAS inhibitor, a significant decrease in mitochondrial membrane potential was exhibited in a control upon treatment with gossypol alone, and it was also confirmed that a significant decrease in mitochondrial membrane potential level was exhibited in an experimental group treated with a mixture of the MAS inhibitor and gossypol (see FIGS. 7A and 7B).

### <4-3> Confirmation of Cell Apoptosis by Co-treatment with MAS inhibitor and Gossypol

To confirm whether a significant synergistic effect on not only the inhibition of cell proliferation but also cell apoptosis can be exhibited upon co-treatment with an MAS inhibitor and gossypol, cell apoptosis activity was examined.

First, A549 cells, UACC62 cells, and IMR90 cells, as a normal control, were cultured. The cultured cells were treated with a drug mixture of a 4 mM SLC25C11 inhibitor (PSA or PA), which is an MAS inhibitor, and 10 μM gossypol or with only one of the drugs and cultured in the same manner as described above for 48 hours. The medium was removed from the cultured cells, followed by washing the cells twice with cold PBS and centrifugation at 1,400 rpm for 3 minutes, and a binding buffer was added thereto so that concentration was 1 x $10^6$ cells/mLdml. Then, a 100 μM buffer was transferred to a 5-mL culture tube and 5 μl of each of Annexin V-FITC and propidium iodine (PI) was added thereto. Mixing was performed by slowly vortexing the tube, and then the cells were incubated in a dark room at room temperature for 15 minutes. After incubation, a binding buffer (400 μl) was added thereto and the degree of increase in cell apoptosis was examined by a flow cytometer.

As a result, as illustrated in FIG. 8, it was confirmed that as a result of culturing cells for 48 hours after treating with a drug, no significant cell apoptosis was exhibited in IMR90 cells (normal control) regardless of treatment with an MAS inhibitor or gossypol alone or co-treatment therewith, whereas cell apoptosis to an insignificant extent was exhibited in A549 cells and UACC62 cells upon treatment with an MAS inhibitor or gossypol alone. However, it was confirmed that the cell apoptosis level was significantly increased upon treatment with a mixture of the MAS inhibitor and gossypol compared to treatment with the MAS inhibitor or gossypol alone (see FIGS. 8A and 8B). Upon treatment with a mixture of an MAS inhibitor and gossypol, not only cell proliferation may be inhibited to a greater extent than when only one of the drugs is used for treatment, but a synergistic effect on cell apoptosis may also be exhibited, thus being effective in cancer cell apoptosis.

### [Example 5] Confirmation of Synergistic Effect on Cancer Cell Proliferation Inhibition through Co-treatment with MAS inhibitor and Phenformin

Since it had been confirmed that a synergistic effect on the inhibition of cancer cell proliferation was exhibited upon co-treatment with an MAS inhibitor and gossypol, thus effectively inhibiting tumor proliferation and effectively inducing cell apoptosis, it was examined whether the MAS inhibitor could also exhibit a synergistic effect with phenformin, which is known as an anticancer agent capable of inducing ATP deficiency and is an inhibitor against mitochondria complex I.

Specifically, A549 cells, UACC62 cells, HOP-62 cells, H226 cells, UACC62 cells, and A375 cells were cultured. 4 mM PSA, 4 mM PTA, or 25 μM to 50 μM NPM was mixed with 100 μM phenformin, and the resulting mixture was added to a culture medium of each cell line, followed by further culturing for 48 hours. Then, cell proliferation levels were examined in the same manner as in Example <1-1> described above, through SRB analysis.

As a result, as illustrated in FIG. 9, it was confirmed that the effect of inhibiting cell proliferation was significantly increased in the case of treatment with a mixture of PSA, PTA, or NPM; and phenformin, as compared to co-treatment with an MAS inhibitor and phenformin (see FIG. 9).

### [Example 6] Confirmation of Synergistic Effect on Cancer Cell Proliferation Inhibition through Co-treatment with MAS inhibitor and Etomoxir

It was examined whether an MAS inhibitor and etomoxir, which is another anticancer agent known to inhibit the proliferation of cancer cells by targeting b-oxidation, could exhibit a synergistic effect. Etomoxir is known as an inhibitor against the activity of an enzyme such as carnitine palmitoyltransferase-1 (CPT-1) located outside the inner mitochondrial membrane, or the like.

Specifically, the liver cancer cell line Huh7, the malignant glioblastoma cell line SNB19, the melanoma cell line UACC62, the breast cancer cell line MDA-MB-231, the gastric cancer cell line KATO III, and IMR90 cells, which are lung fibroblasts and a normal control, were separately cultured. 25 μM NPM and 100 μM etomoxir were mixed, and the resulting mixture was added to a culture medium of each cell line, followed by further culturing for 24 hours. Then, cell proliferation levels were examined in the same manner as in Example <1-1> described above, through SRB analysis.

As a result, as illustrated in FIG. 10, it was confirmed that the effect of inhibiting cancer cell proliferation could be exhibited in the cancer cell lines other than the malignant glioblastoma cell line SNB19 upon treatment with NPM, which is an MAS inhibitor. It was also confirmed that the effect of inhibiting cell proliferation was significantly increased upon co-treatment with a mixture of etomoxir and NPM compared to an experimental group treated with etomoxir or NPM alone, and that, in the case of the lung cancer cell line Huh7, not only cell proliferation was inhibited, but cell apoptosis was also exhibited, resulting in a decrease in the number of cancer cell lines (see FIG. 10).

## Claims

1. A pharmaceutical composition for preventing or treating cancer, the pharmaceutical composition comprising a malate-aspartate shuttle (MAS) inhibitor as an active ingredient.

2. The pharmaceutical composition of claim 1, wherein the MAS inhibitor is an inhibitor against the expression or activity of a protein contained in MAS.

3. The pharmaceutical composition of claim 2, wherein the protein contained in MAS comprises any one or more selected from the group consisting of malate-$\alpha$-ketoglutarate transporter (MAT), glutamate-aspartate transporter (GAT), malate dehydrogenase 1, malate dehydrogenase 2, glutamic oxaloacetic transaminase 1, and glutamic oxaloacetic transaminase 2.

4. The pharmaceutical composition of claim 2, wherein the inhibitor against the expression of the protein contained in MAS comprises at least one of siRNA and shRNA against the protein contained in MAS.

5. The pharmaceutical composition of claim 4, wherein the siRNA is any one of a forward siRNA having a nucleotide sequence represented by SEQ ID NO: 1 and a reverse siRNA having a nucleotide sequence represented by SEQ ID NO: 2; and a forward siRNA having a nucleotide sequence represented by SEQ ID NO: 3 and a reverse siRNA having a nucleotide sequence represented by SEQ ID NO: 4, and the shRNA is any one of a nucleotide sequence represented by SEQ ID NO: 5 and a nucleotide sequence represented by SEQ ID NO: 6.

6. The pharmaceutical composition of claim 2, wherein the inhibitor against the activity of the protein contained in MAS comprises any one or more selected from the group consisting of phenyl succinic acid, methyl malonic acid, N-(1-pyrenyl)maleimide, phthalonic acid, methyl 3-(3-(4-(2,4,4-trimethylpentan-2-yl)phenoxy)-propanamido)benzoate), LW6, 2-thenoyl-trifluoroacetone, chlorothricin, aminooxyacetic acid (AOA), hydrazinosuccinate, 2-amino-3-butenoic acid, and a mercurial reagent.

7. The pharmaceutical composition of claim 1, wherein the cancer comprises any one or more selected from the group consisting of lung cancer, melanoma, uterine cancer, breast cancer, gastric cancer, brain cancer, rectal cancer, colon cancer, skin cancer, blood cancer, liver cancer, ovarian cancer, kidney cancer, prostate cancer, and pancreatic cancer.

8. A pharmaceutical composition for preventing or treating cancer, the pharmaceutical composition comprising the pharmaceutical composition of claim 1 and an anticancer agent.

**9.** The pharmaceutical composition of claim 8, wherein the anticancer agent comprises any one or more selected from the group consisting of gossypol, phenformin, and etomoxir.

**10.** The pharmaceutical composition of claim 8, wherein in the pharmaceutical composition for preventing or treating cancer, the anticancer agent and the MAS inhibitor are mixed in a molar ratio of 1:10 to 500.

**11.** The pharmaceutical composition of claim 8, wherein the cancer comprises any one or more selected from the group consisting of lung cancer, melanoma, uterine cancer, breast cancer, gastric cancer, brain cancer, rectal cancer, colon cancer, skin cancer, blood cancer, liver cancer, ovarian cancer, kidney cancer, prostate cancer, and pancreatic cancer.

**12.** A method of treating cancer, the method comprising administering an effective amount of a malate-aspartate shuttle inhibitor to an individual in need thereof.

**13.** A method of treating cancer, the method comprising administering effective amounts of an MAS inhibitor and an anticancer agent to an individual in need thereof.

**14.** A use of a pharmaceutical composition for preventing or treating cancer, the pharmaceutical composition comprising a malate-aspartate shuttle inhibitor.

**15.** A use of a pharmaceutical composition for preventing or treating cancer, the pharmaceutical composition comprising a malate-aspartate shuttle inhibitor and an anticancer agent.

Figure 1A

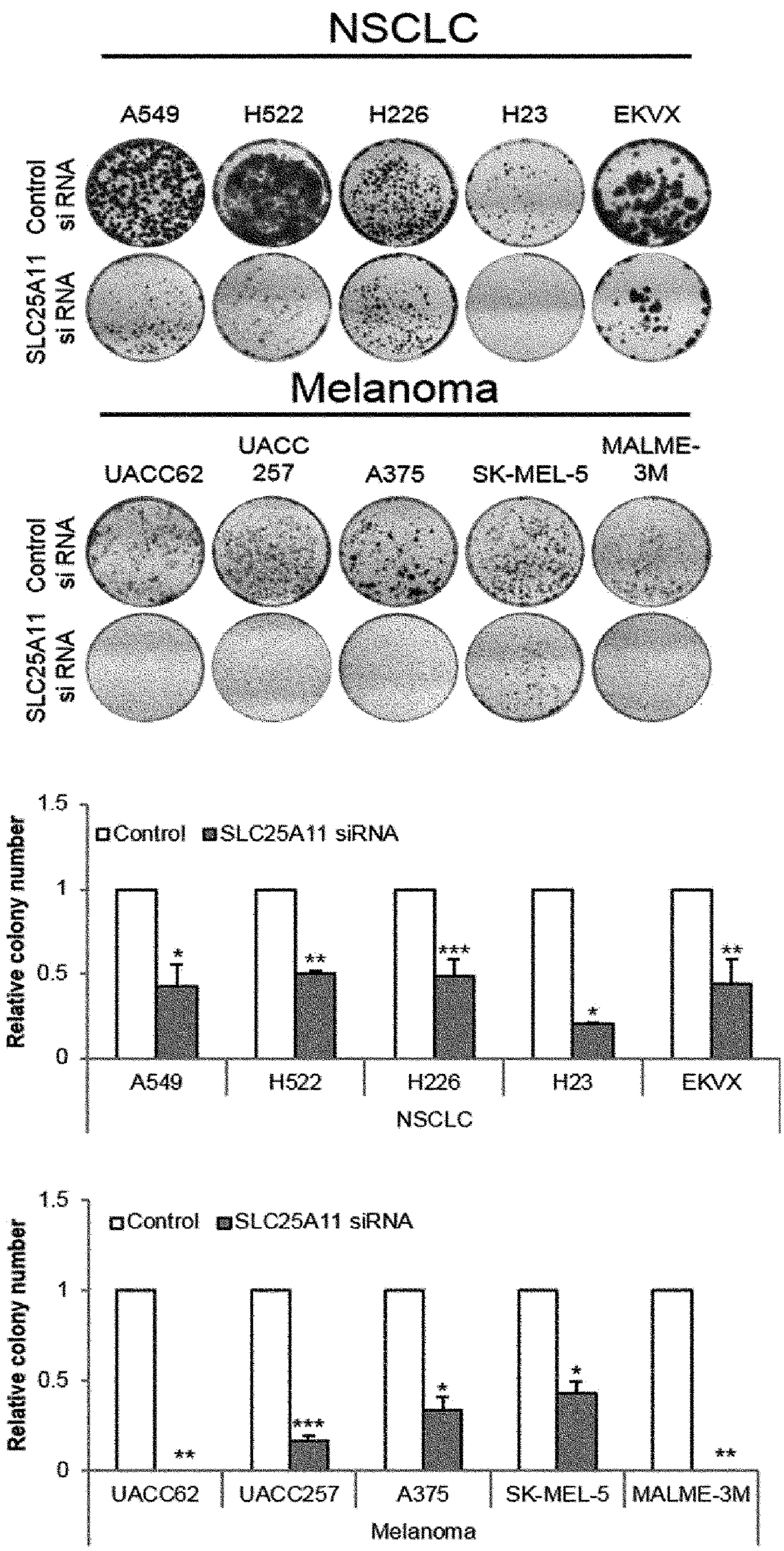
NSCLC
A549
H522
H226
H23
EKVX
Control si RNA
SLC25A11 si RNA
Melanoma
UACC62
UACC 257
A375
SK-MEL-5
MALME-3M
Control si RNA
SLC25A11 si RNA
Relative colony number
Control
SLC25A11 siRNA
1.5
1
0.5
0
*
**
***
*
**
A549
H522
H226
H23
EKVX
NSCLC
Relative colony number
Control
SLC25A11 siRNA
1.5
1
0.5
0
**
***
*
*
**
UACC62
UACC257
A375
SK-MEL-5
MALME-3M
Melanoma

Figure 1B

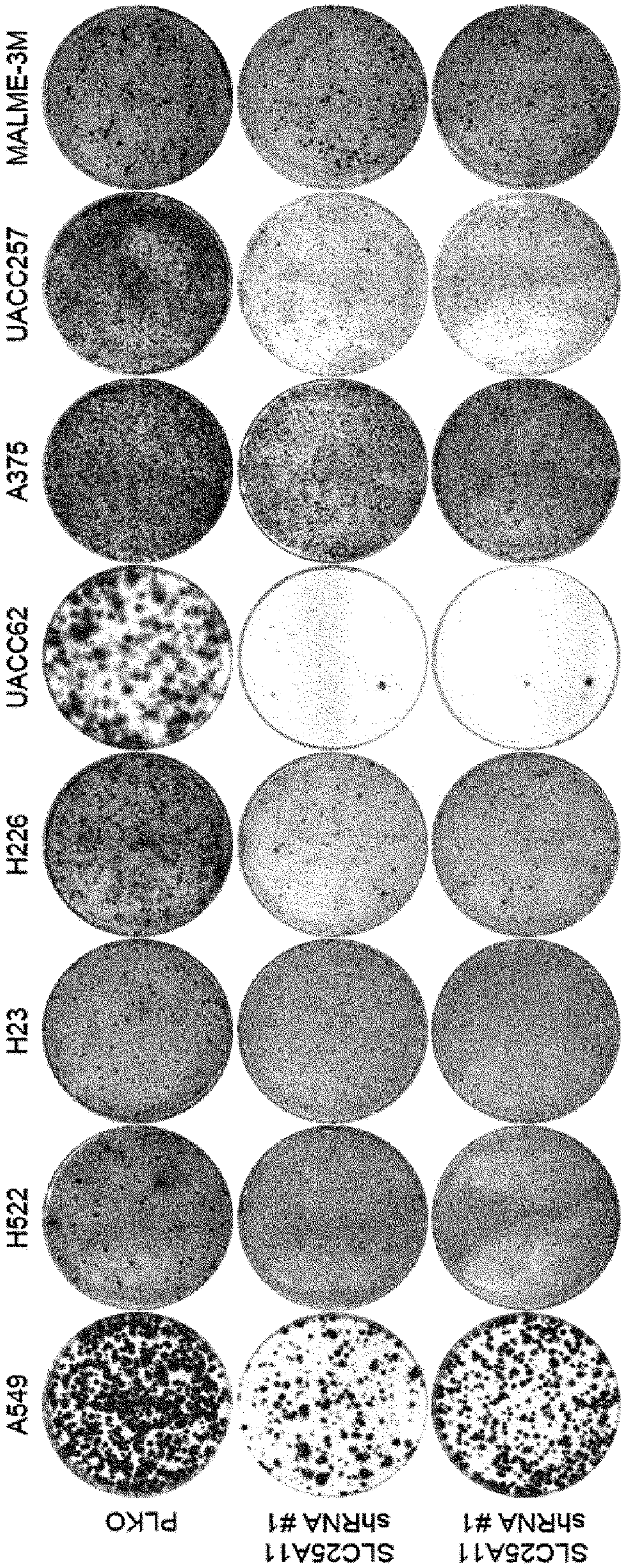
MALME-3M
UACC257
A375
UACC62
H226
H23
H522
A549
PLKO
SLC25A11 shRNA #1
SLC25A11 shRNA #1

Figure 1C

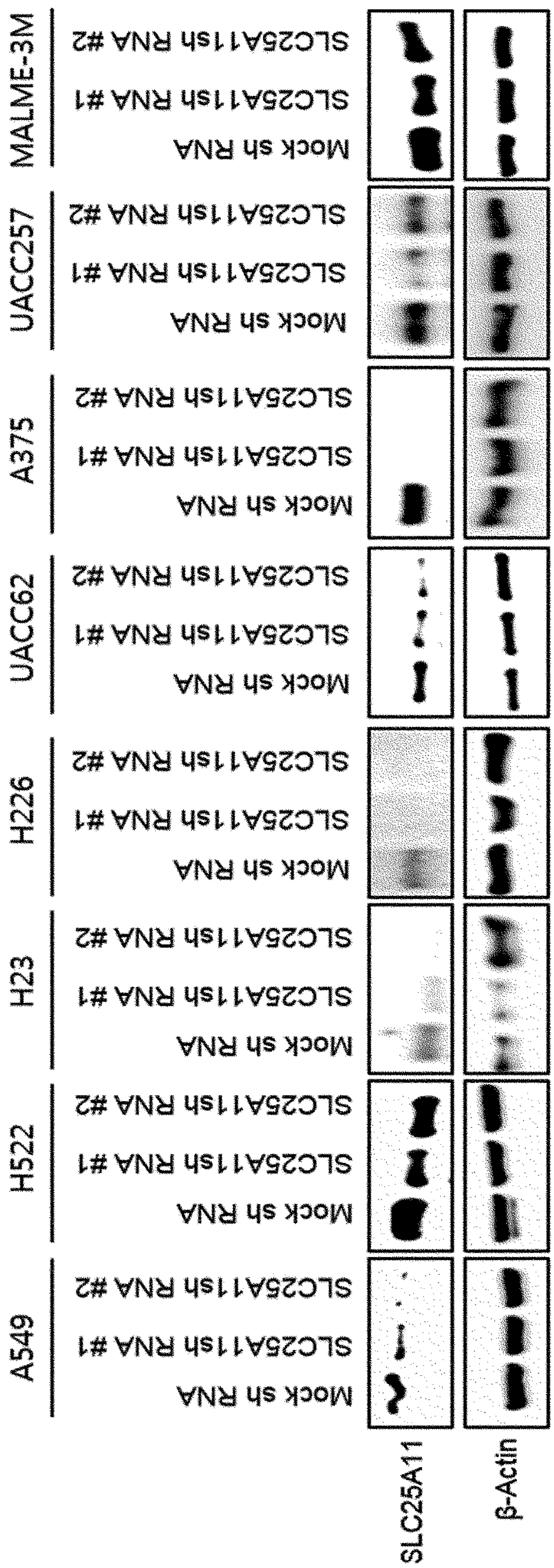
MALME-3M
UACC257
A375
UACC62
H226
H23
H522
A549
Mock sh RNA
SLC25A11sh RNA #1
SLC25A11sh RNA #2
SLC25A11
β-Actin

Figure 2A

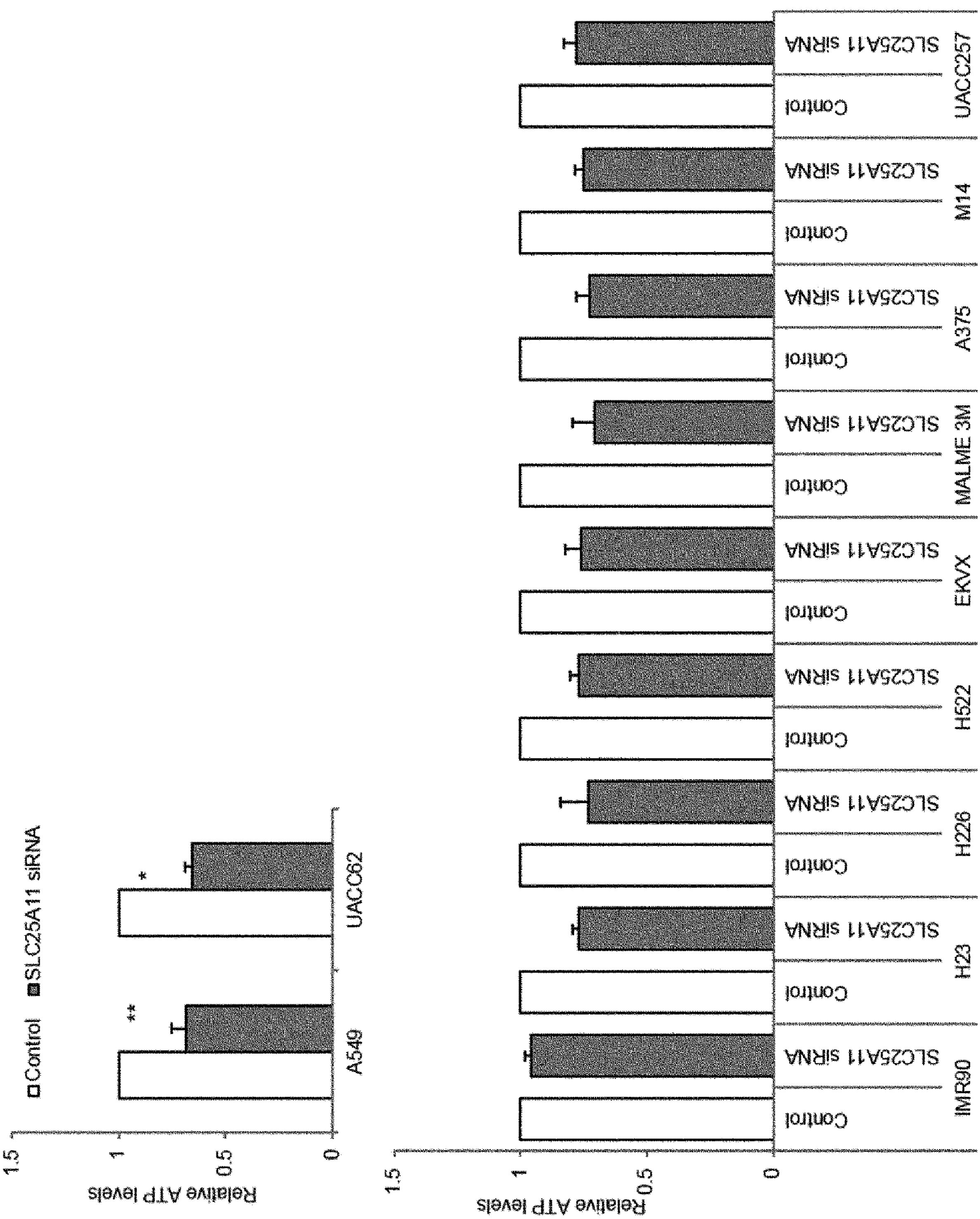
Control
SLC25A11 siRNA
Relative ATP levels
1.5
1
0.5
0
A549
UACC62
**
*
IMR90
H23
H226
H522
EKVX
MALME 3M
A375
M14
UACC257

Figure 2B

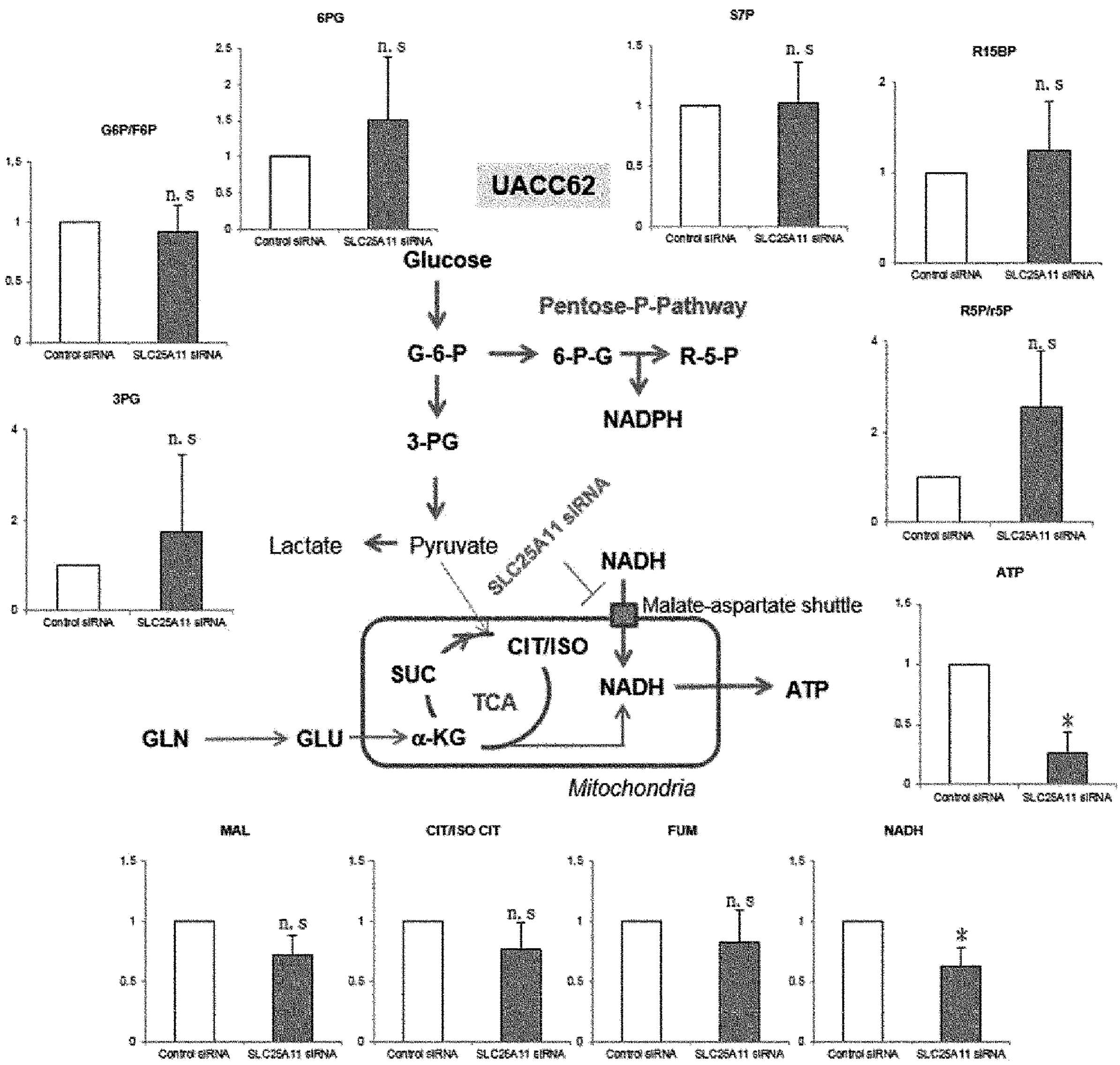
UACC62
Glucose
G-6-P
6-P-G
R-5-P
Pentose-P-Pathway
NADPH
3-PG
Pyruvate
Lactate
SLC25A11 siRNA
NADH
Malate-aspartate shuttle
CIT/ISO
SUC
TCA
α-KG
NADH
ATP
GLN
GLU
Mitochondria
G6P/F6P
6PG
S7P
R15BP
R5P/r5P
3PG
ATP
MAL
CIT/ISO CIT
FUM
NADH
Control siRNA
SLC25A11 siRNA
n. s
*

Figure 3A

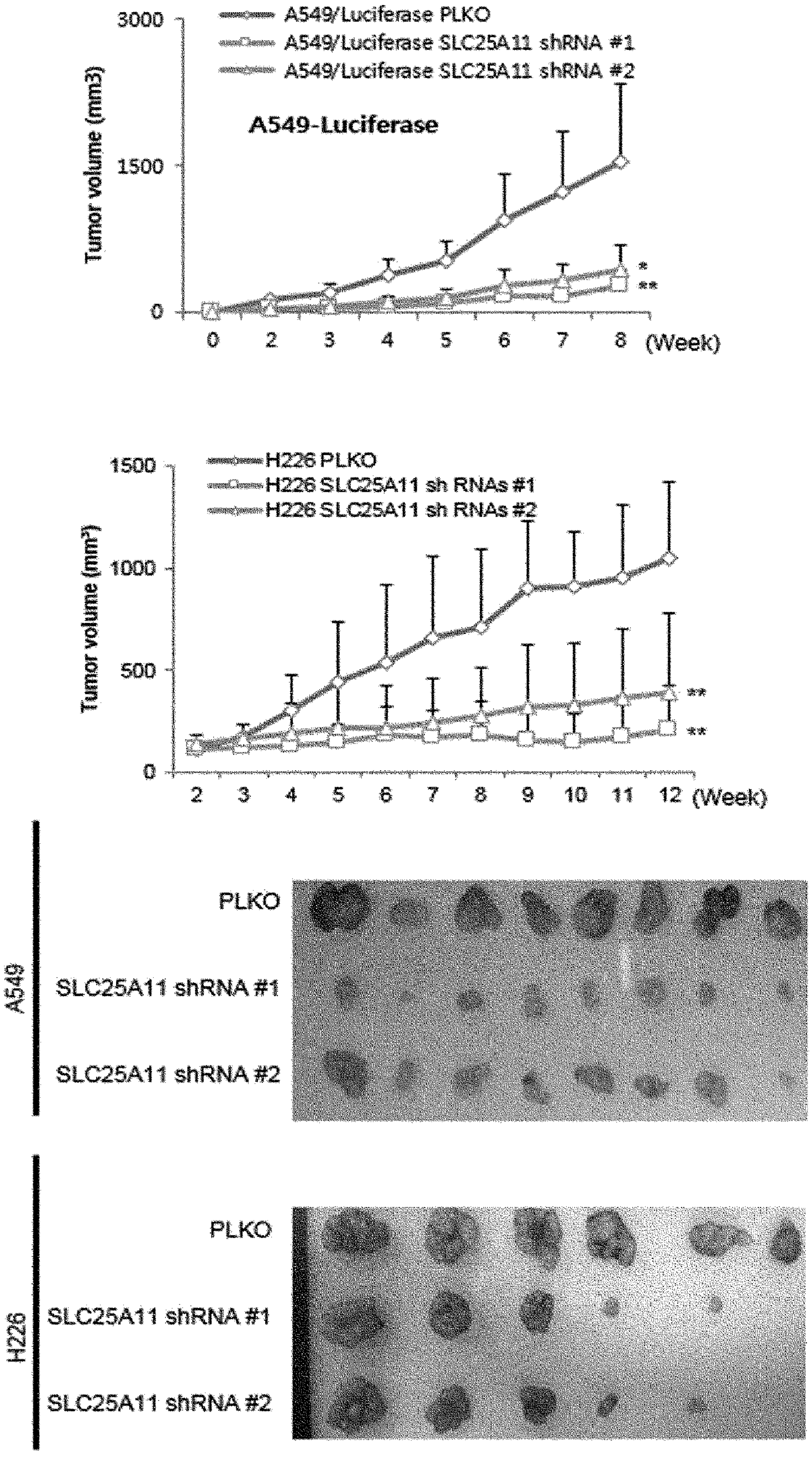
A549/Luciferase PLKO
A549/Luciferase SLC25A11 shRNA #1
A549/Luciferase SLC25A11 shRNA #2
A549-Luciferase
Tumor volume (mm3)
3000
1500
0
0 2 3 4 5 6 7 8 (Week)
*
**
H226 PLKO
H226 SLC25A11 sh RNAs #1
H226 SLC25A11 sh RNAs #2
Tumor volume (mm³)
1500
1000
500
0
2 3 4 5 6 7 8 9 10 11 12 (Week)
**
**
A549
PLKO
SLC25A11 shRNA #1
SLC25A11 shRNA #2
H226
PLKO
SLC25A11 shRNA #1
SLC25A11 shRNA #2

Figure 3B

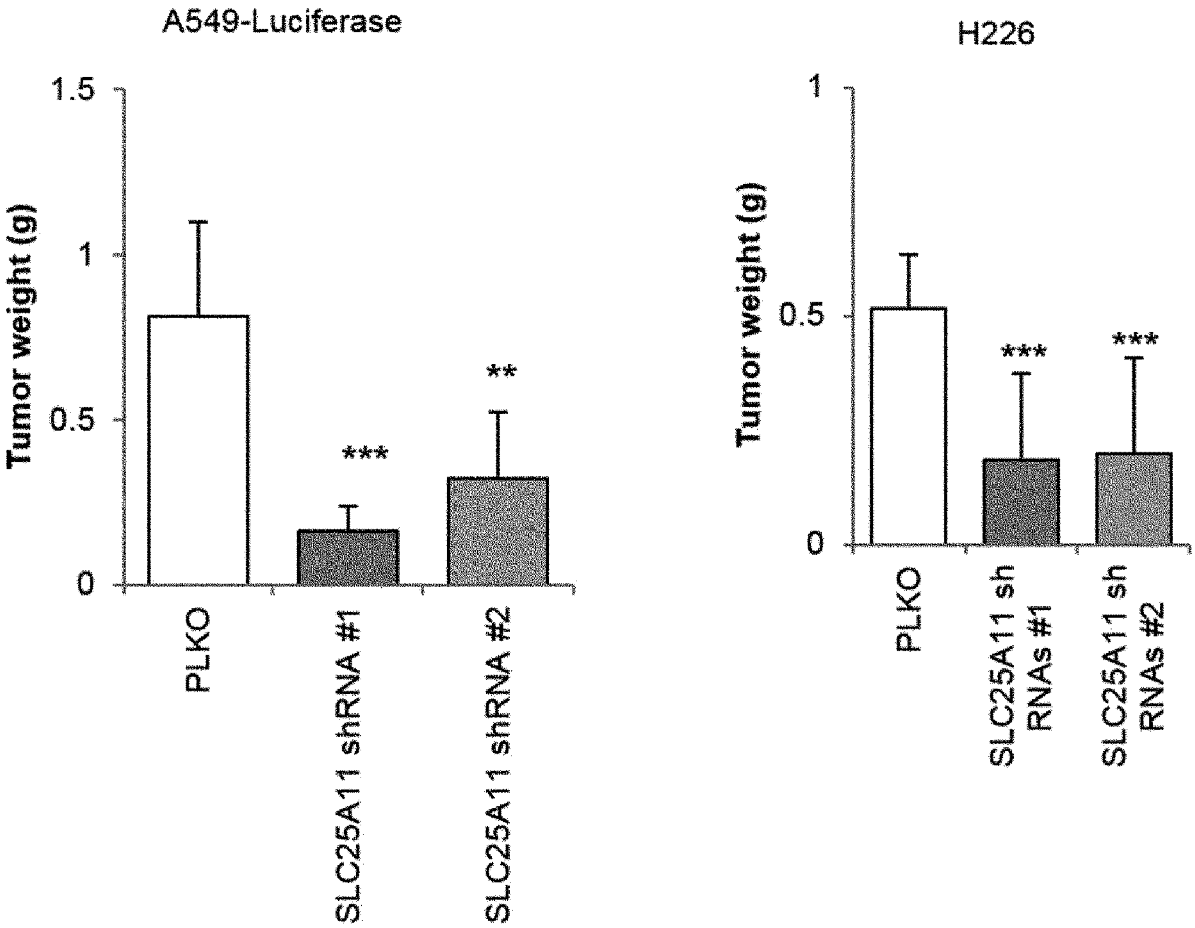
A549-Luciferase
Tumor weight (g)
1.5
1
0.5
0
***
**
PLKO
SLC25A11 shRNA #1
SLC25A11 shRNA #2
H226
Tumor weight (g)
1
0.5
0
***
***
PLKO
SLC25A11 sh RNAs #1
SLC25A11 sh RNAs #2

Figure 4

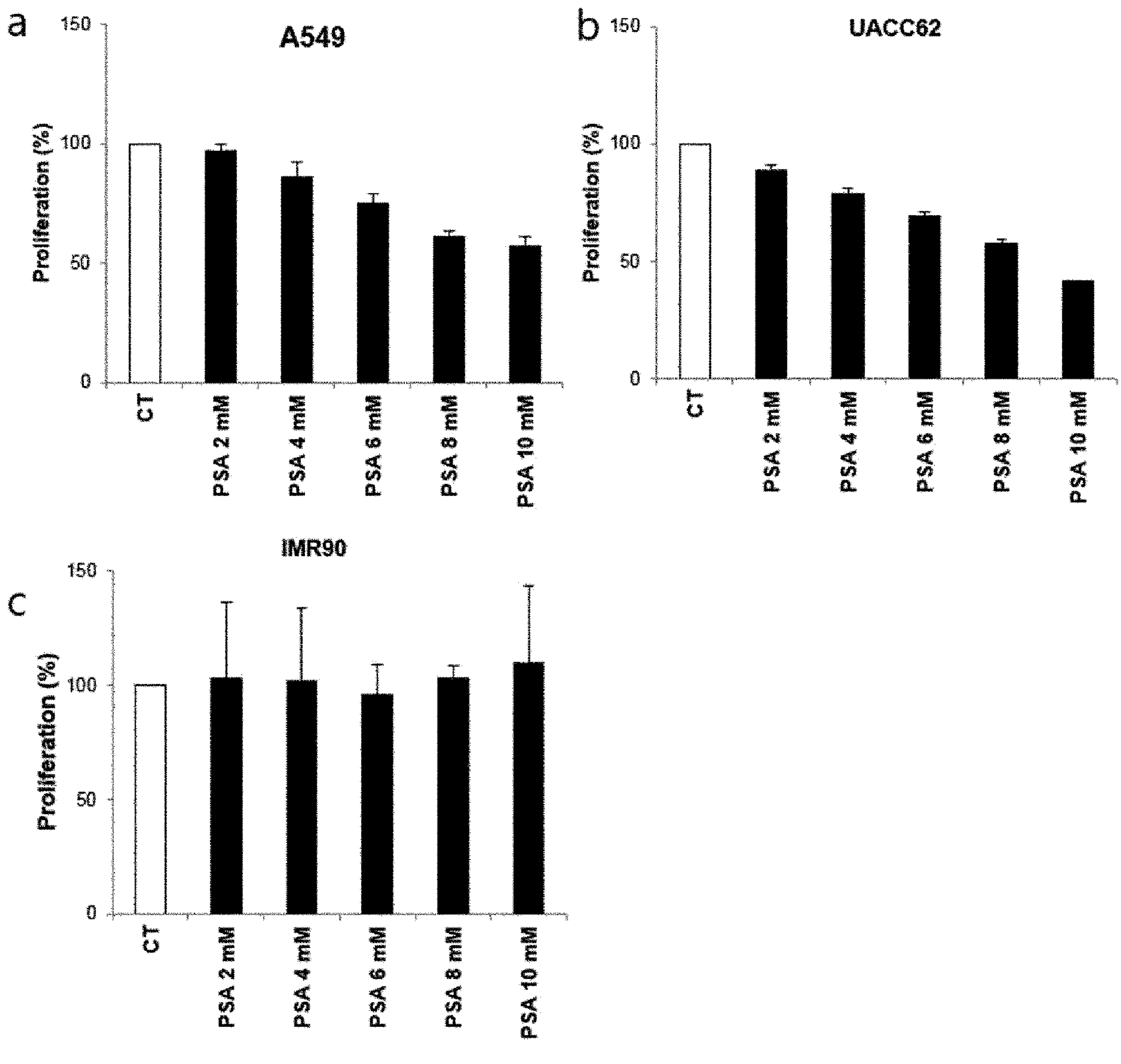
a
A549
150
100
50
0
Proliferation (%)
CT
PSA 2 mM
PSA 4 mM
PSA 6 mM
PSA 8 mM
PSA 10 mM
b
UACC62
150
100
50
0
Proliferation (%)
CT
PSA 2 mM
PSA 4 mM
PSA 6 mM
PSA 8 mM
PSA 10 mM
c
IMR90
150
100
50
0
Proliferation (%)
CT
PSA 2 mM
PSA 4 mM
PSA 6 mM
PSA 8 mM
PSA 10 mM

Figure 5

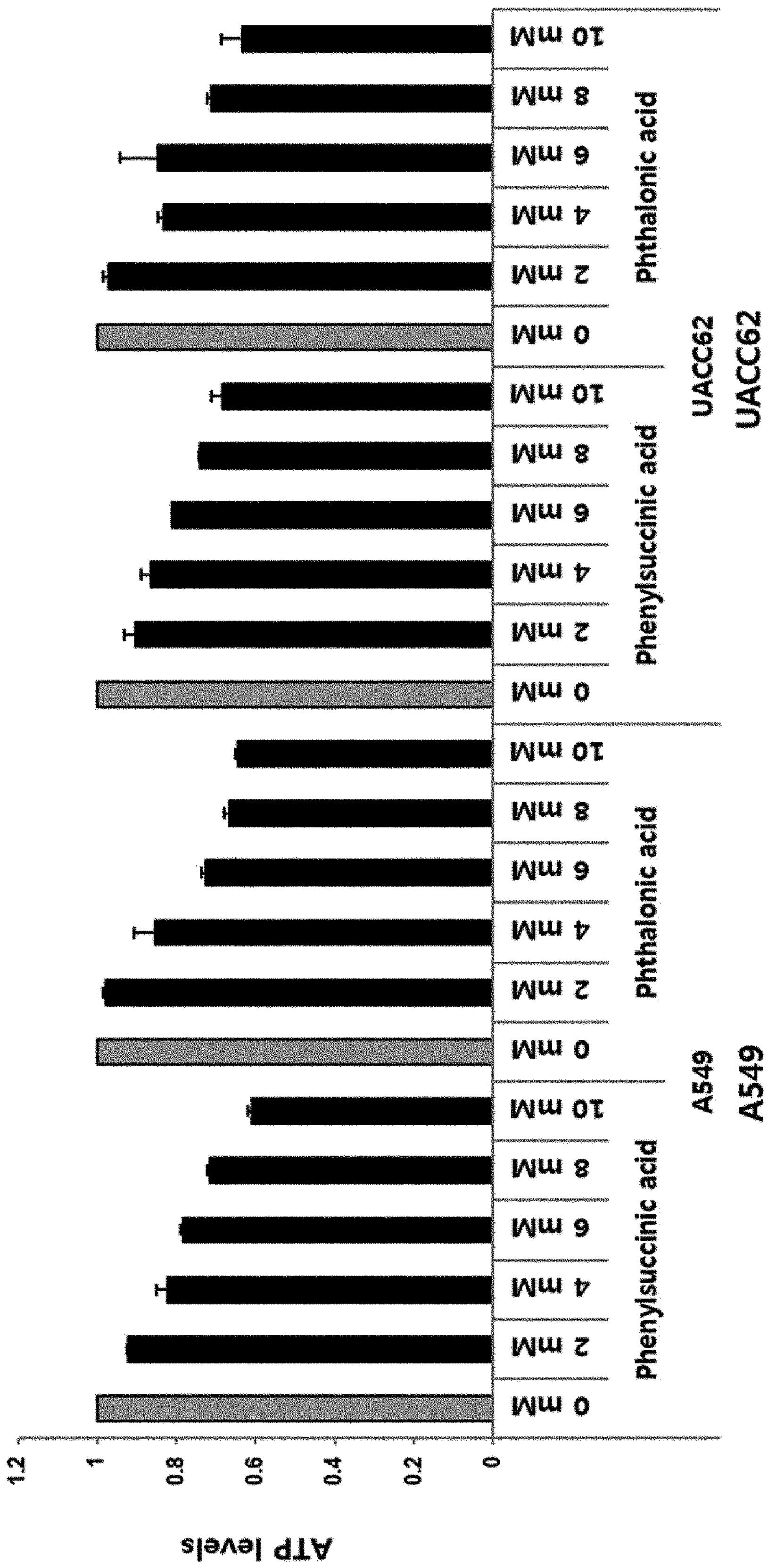
ATP levels
1.2
1
0.8
0.6
0.4
0.2
0
0 mM
2 mM
4 mM
6 mM
8 mM
10 mM
Phenylsuccinic acid
Phthalonic acid
A549
UACC62

Figure 6A

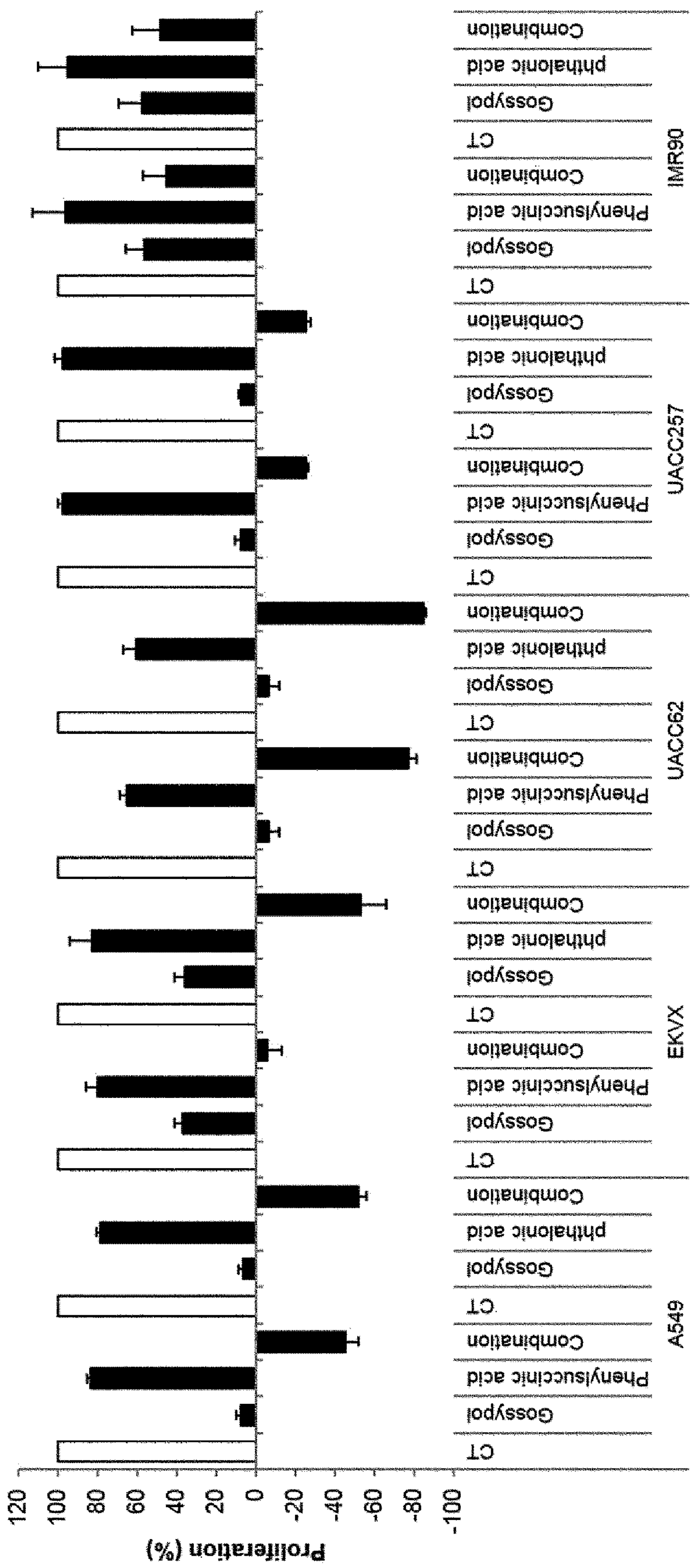
Proliferation (%)
120
100
80
60
40
20
0
-20
-40
-60
-80
-100
CT
Gossypol
Phenylsuccinic acid
Combination
phthalonic acid
A549
EKVX
UACC62
UACC257
IMR90

Figure 6B

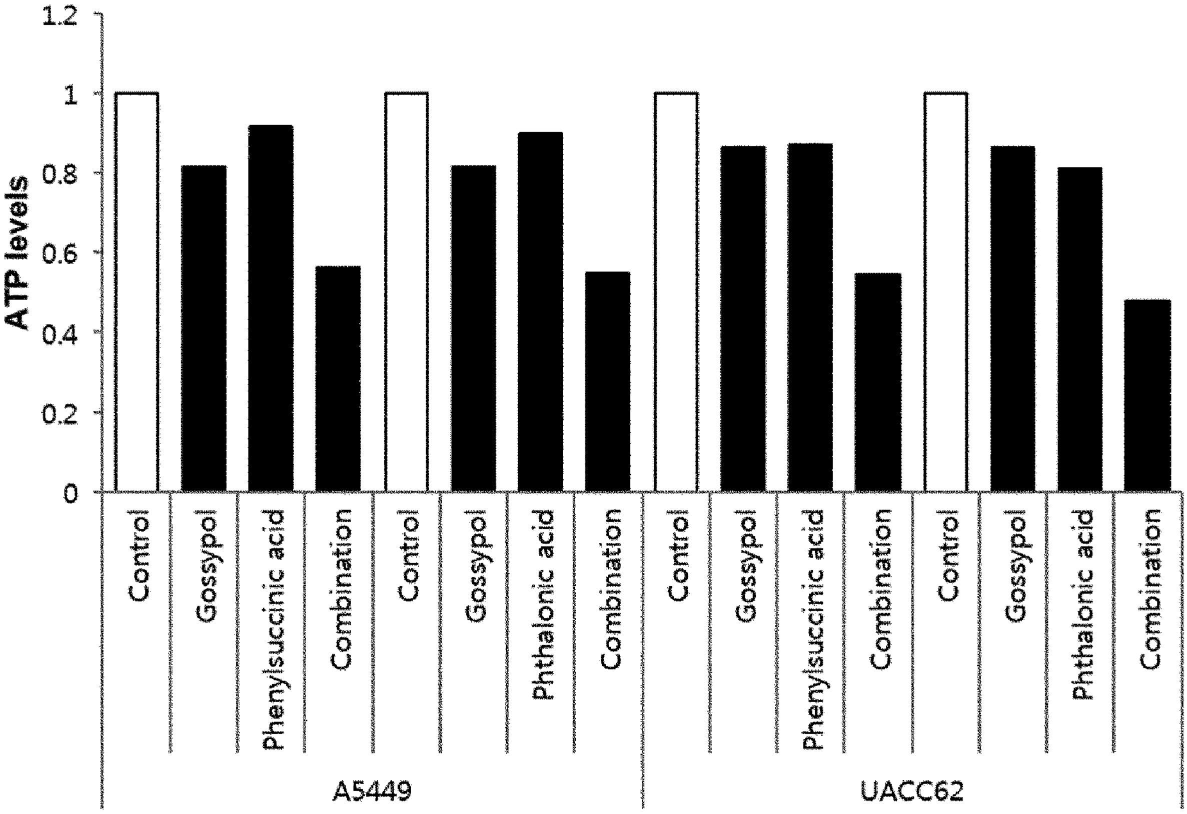
1.2
1
0.8
0.6
0.4
0.2
0
ATP levels
Control
Gossypol
Phenylsuccinic acid
Combination
Control
Gossypol
Phthalonic acid
Combination
Control
Gossypol
Phenylsuccinic acid
Combination
Control
Gossypol
Phthalonic acid
Combination
A5449
UACC62

Figure 6C

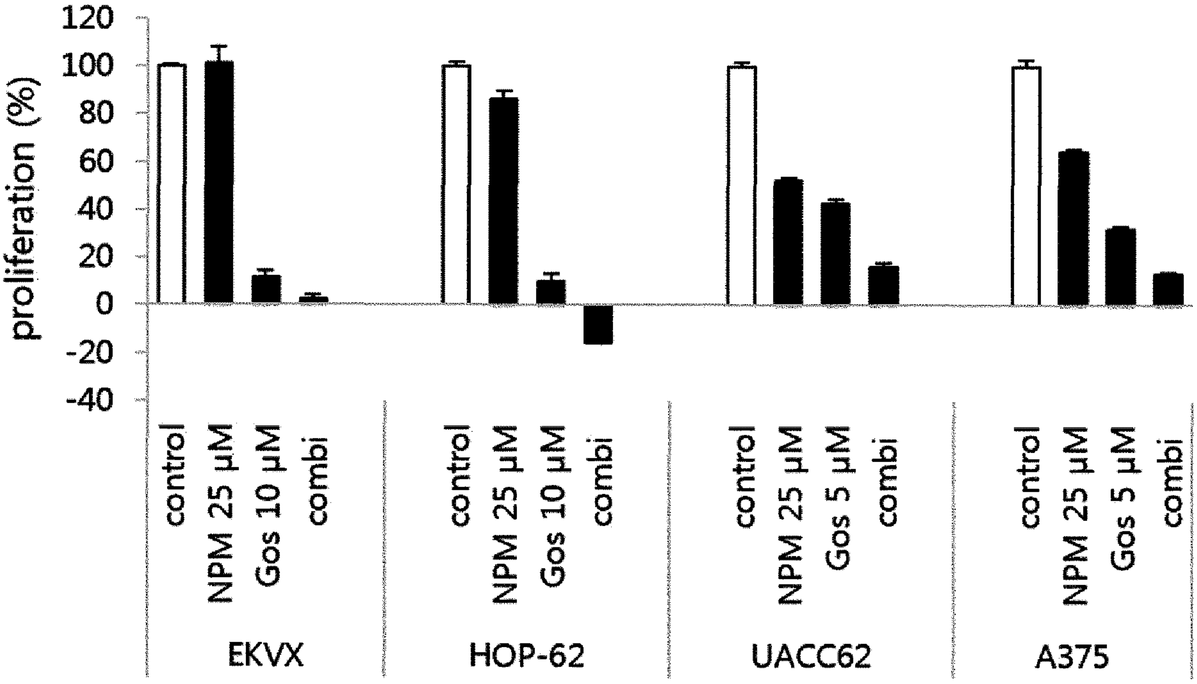


NPM: N-(1-Pyrenyl) maleimide
Gos: Gossypol

Figure 7A

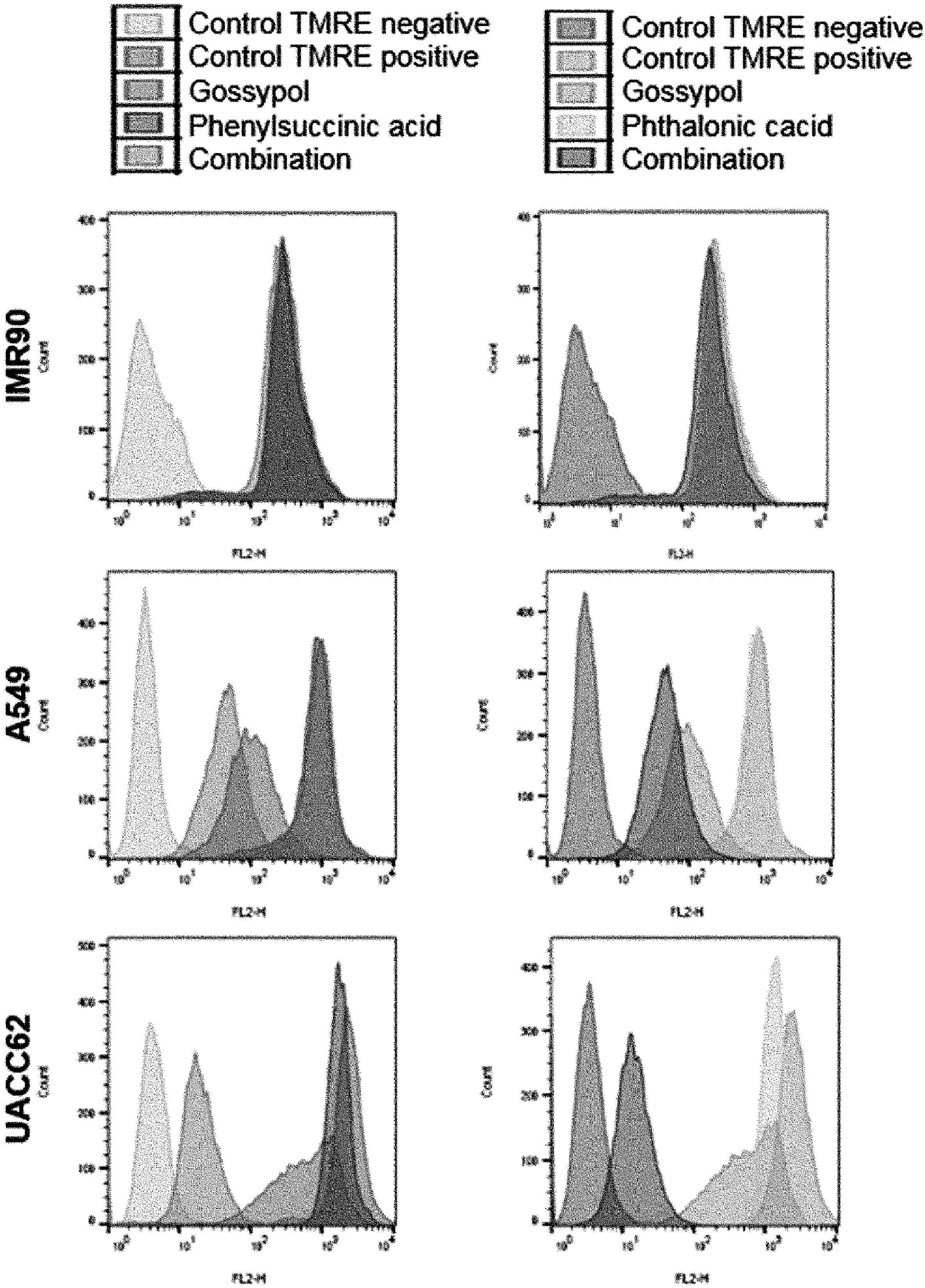
Control TMRE negative
Control TMRE positive
Gossypol
Phenylsuccinic acid
Combination
Control TMRE negative
Control TMRE positive
Gossypol
Phthalonic cacid
Combination
IMR90
A549
UACC62
Count
FL2-H

Figure 7B

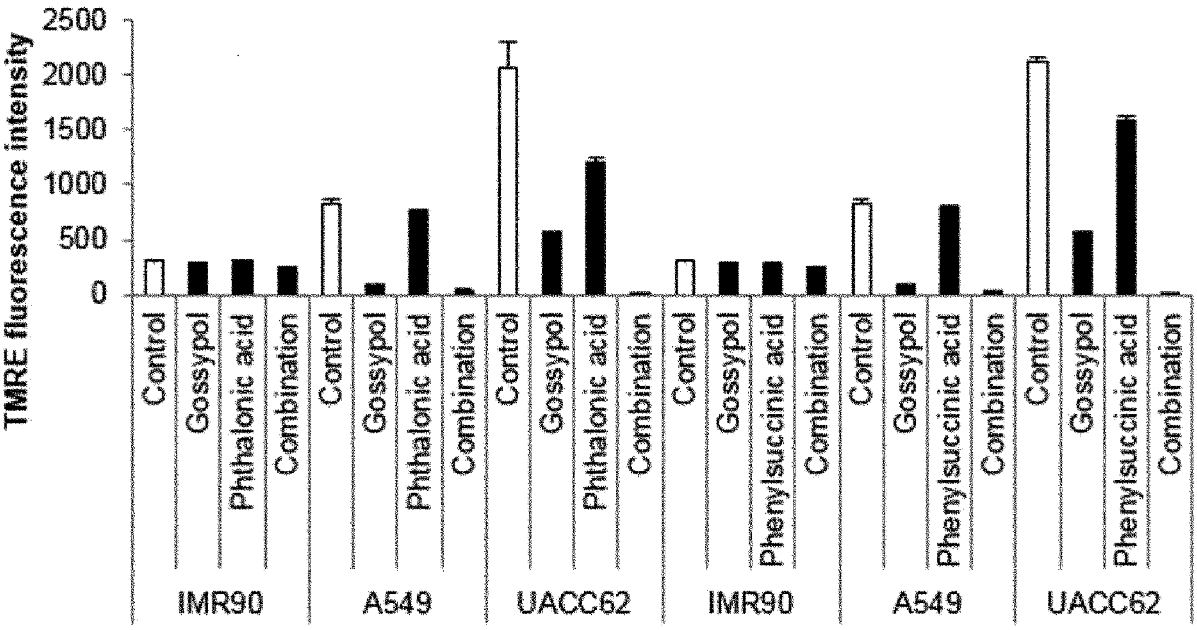
TMRE fluorescence intensity
2500
2000
1500
1000
500
0
Control
Gossypol
Phthalonic acid
Combination
Control
Gossypol
Phthalonic acid
Combination
Control
Gossypol
Phthalonic acid
Combination
Control
Gossypol
Phenylsuccinic acid
Combination
Control
Gossypol
Phenylsuccinic acid
Combination
Control
Gossypol
Phenylsuccinic acid
Combination
IMR90
A549
UACC62
IMR90
A549
UACC62

Figure 8A

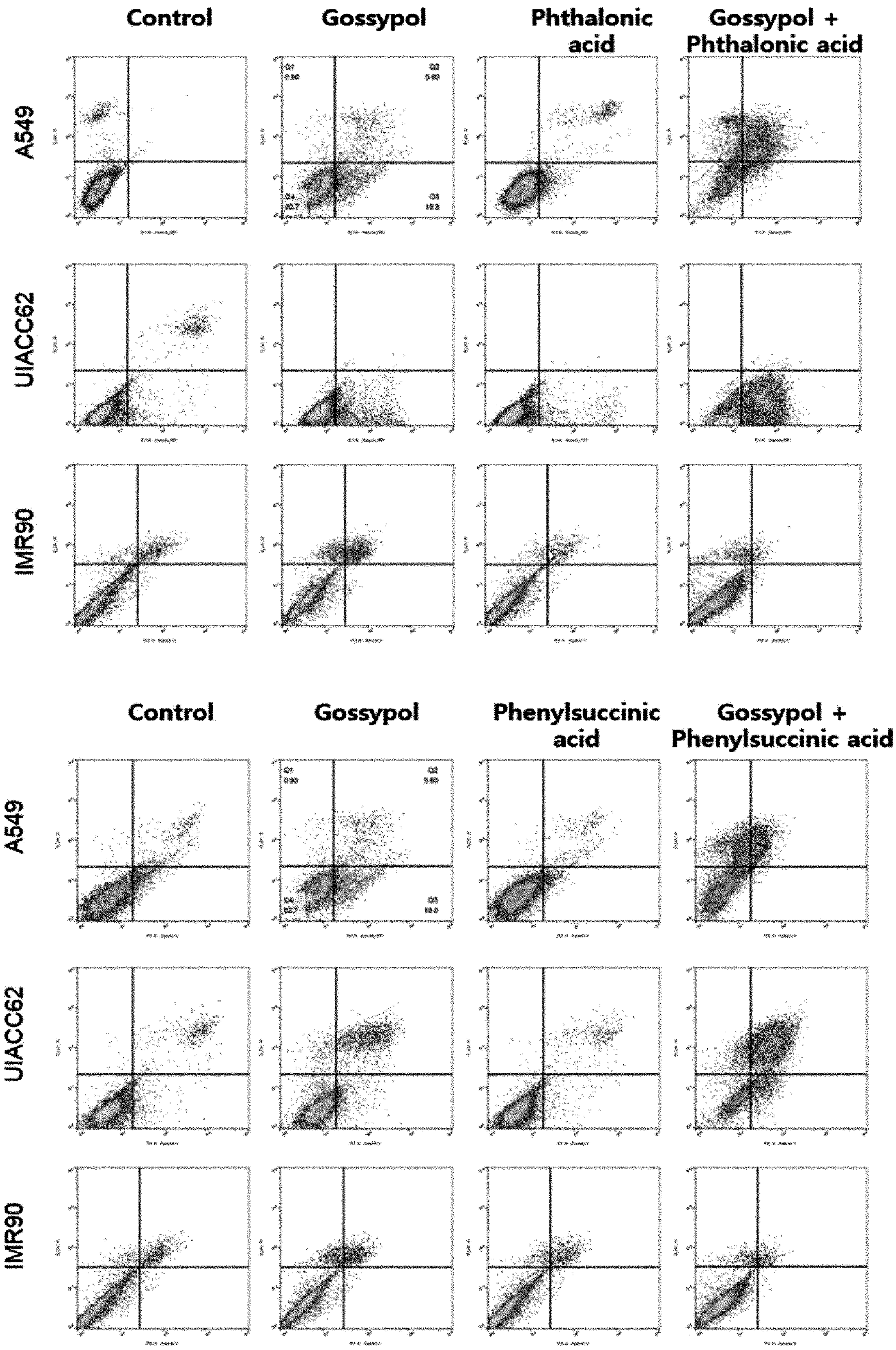
Control
Gossypol
Phthalonic acid
Gossypol + Phthalonic acid
A549
UIACC62
IMR90
Control
Gossypol
Phenylsuccinic acid
Gossypol + Phenylsuccinic acid
A549
UIACC62
IMR90

Figure 8B

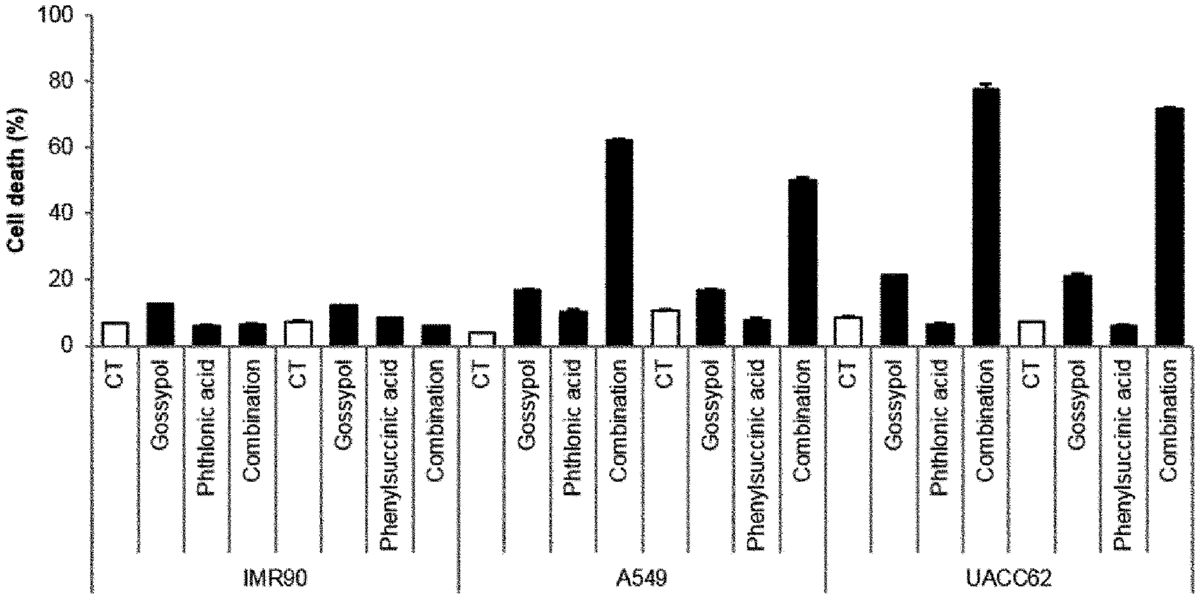
100
80
60
40
20
0
Cell death (%)
CT
Gossypol
Phthlonic acid
Combination
CT
Gossypol
Phenylsuccinic acid
Combination
CT
Gossypol
Phthlonic acid
Combination
CT
Gossypol
Phenylsuccinic acid
Combination
CT
Gossypol
Phthlonic acid
Combination
CT
Gossypol
Phenylsuccinic acid
Combination
IMR90
A549
UACC62

Figure 9A

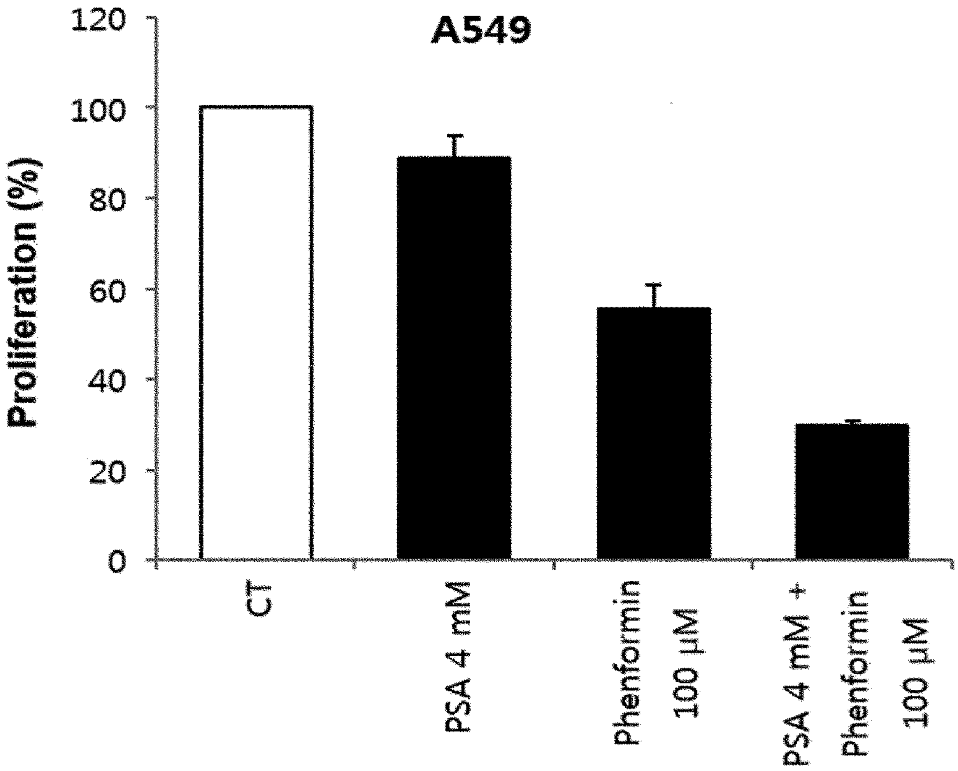
A549
Proliferation (%)
120
100
80
60
40
20
0
CT
PSA 4 mM
Phenformin 100 µM
PSA 4 mM + Phenformin 100 µM

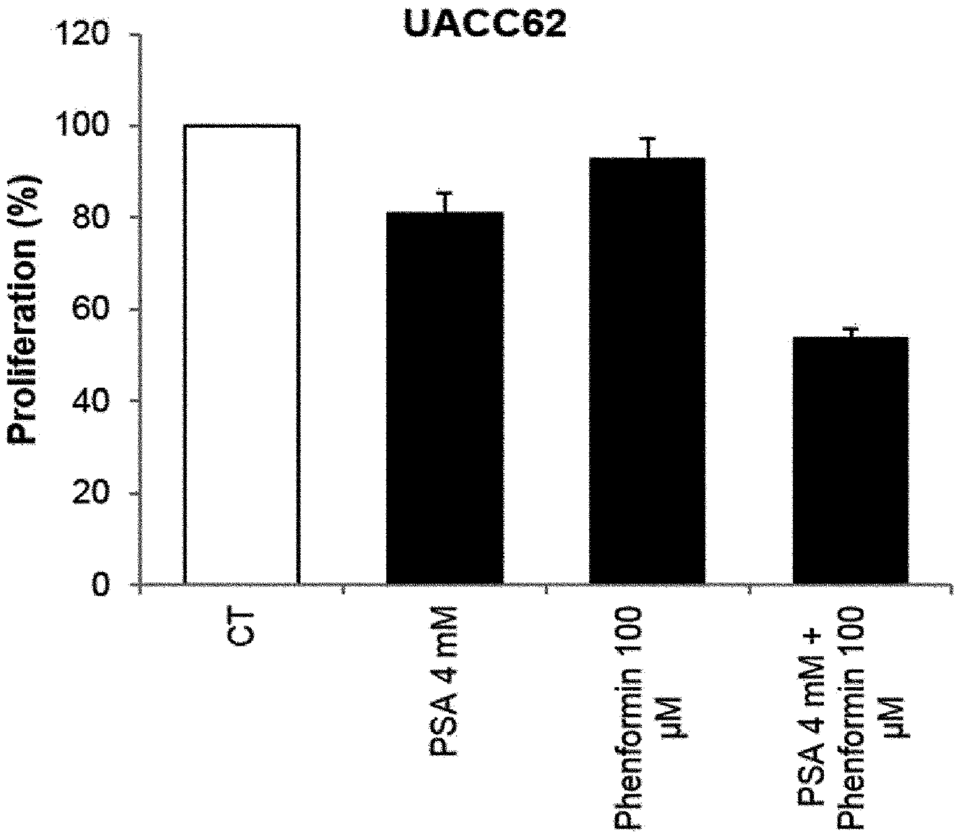
UACC62
Proliferation (%)
120
100
80
60
40
20
0
CT
PSA 4 mM
Phenformin 100 µM
PSA 4 mM + Phenformin 100 µM

Figure 9B

A549

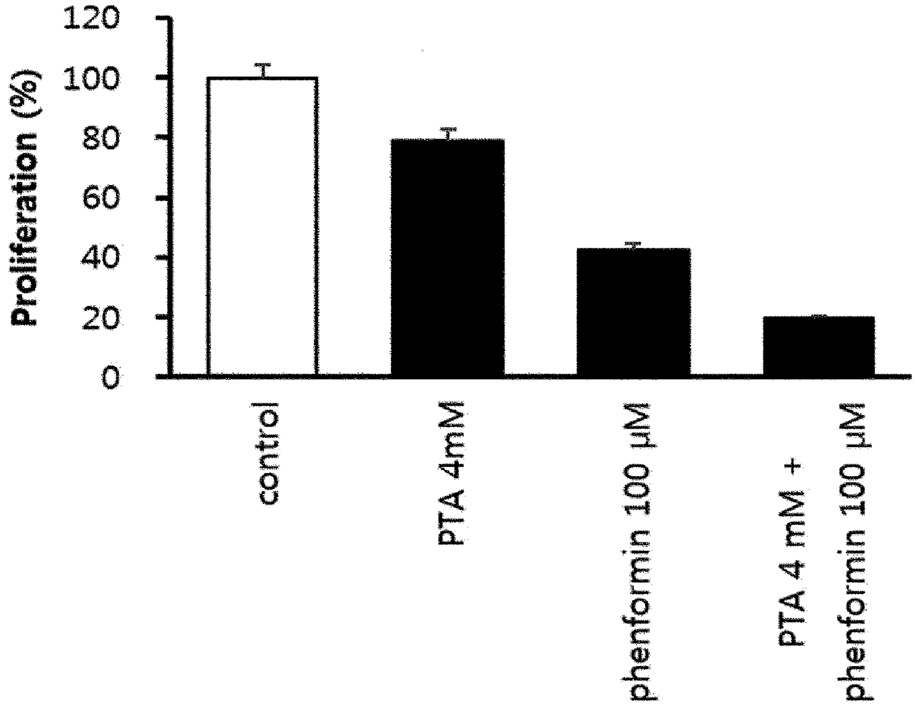


PTA: phthalonic acid

UACC62

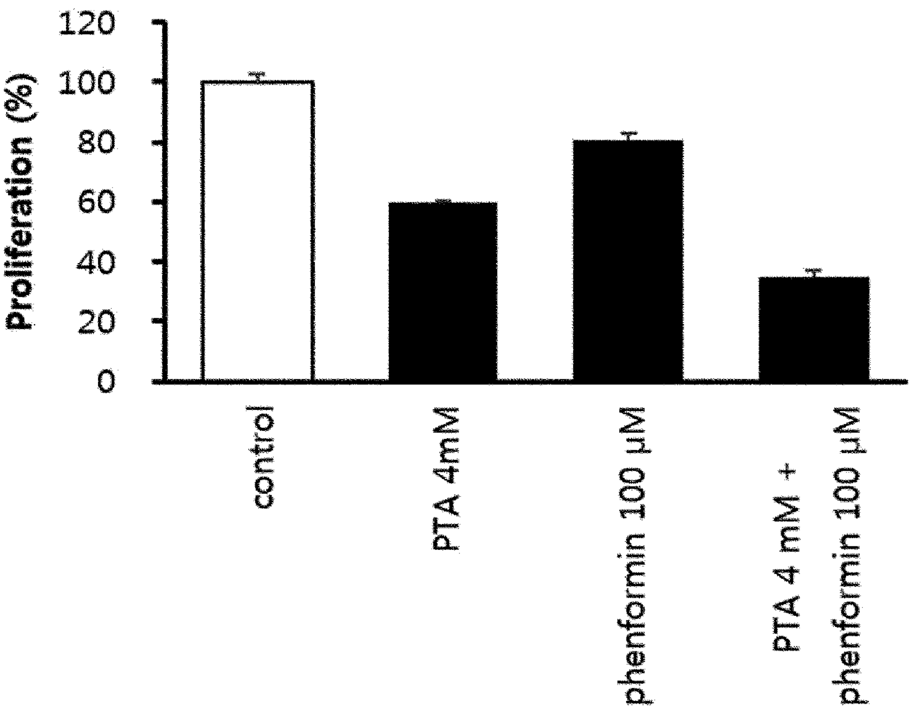

Figure 9C

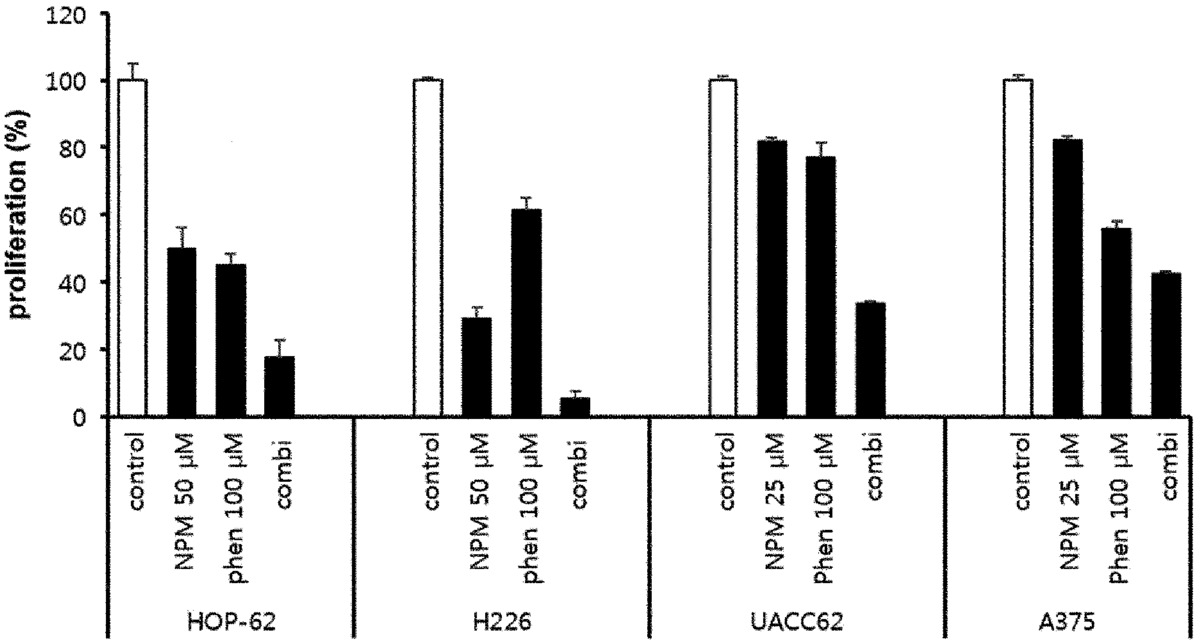
proliferation (%)
120
100
80
60
40
20
0
control
NPM 50 µM
phen 100 µM
combi
HOP-62
control
NPM 50 µM
phen 100 µM
combi
H226
control
NPM 25 µM
Phen 100 µM
combi
UACC62
control
NPM 25 µM
Phen 100 µM
combi
A375

Figure 10

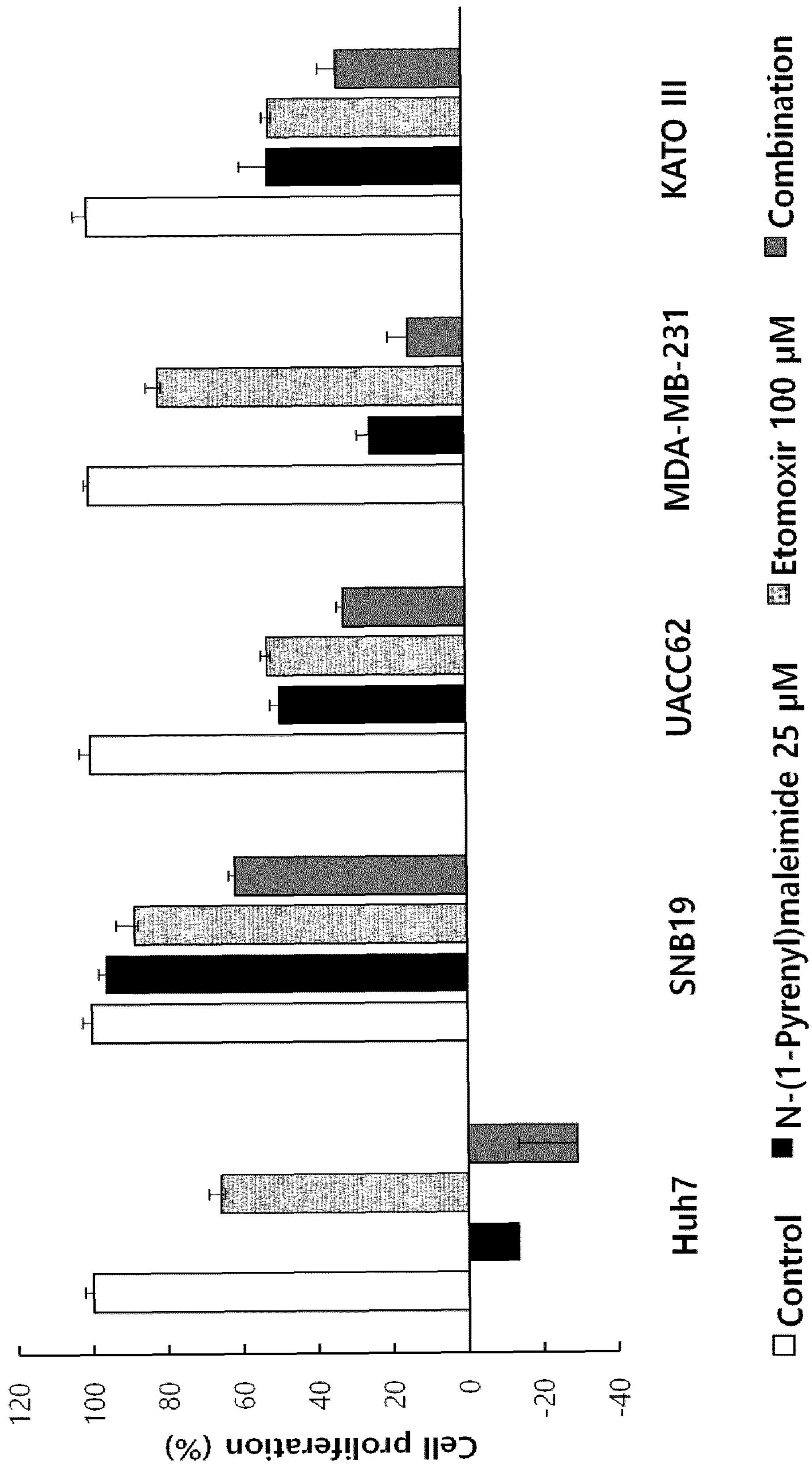

Cell proliferation (%)
120
100
80
60
40
20
0
-20
-40
Huh7
SNB19
UACC62
MDA-MB-231
KATO III
Control
N-(1-Pyrenyl)maleimide 25 µM
Etomoxir 100 µM
Combination

Figure 11

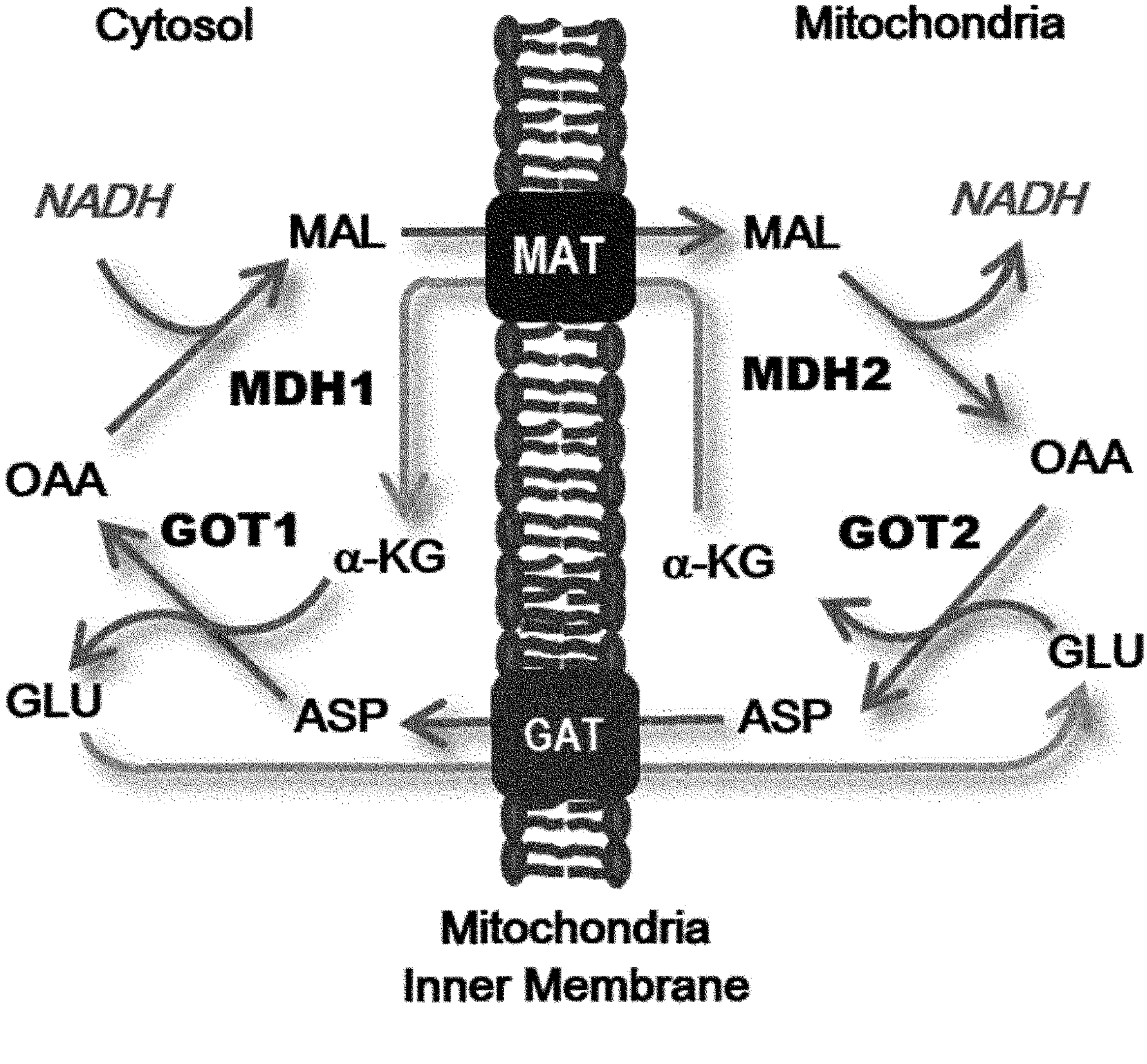
Cytosol
Mitochondria
NADH
MAL
MAT
MAL
NADH
MDH1
MDH2
OAA
OAA
GOT1
α-KG
α-KG
GOT2
GLU
GLU
ASP
GAT
ASP
Mitochondria
Inner Membrane

**INTERNATIONAL SEARCH REPORT**

International application No.
**PCT/KR2018/004344**

A. CLASSIFICATION OF SUBJECT MATTER

*A61K 31/194(2006.01)i, A61K 31/11(2006.01)i, A61K 31/155(2006.01)i*

According to International Patent Classification (IPC) or to both national classification and IPC

B. FIELDS SEARCHED

Minimum documentation searched (classification system followed by classification symbols)
A61K 31/194; C07H 21/02; A61K 31/475; A61K 48/00; G01N 33/50; A61K 31/11; A61K 31/155

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: malate-aspartate shuttle inhibitor, anticancer, gossypol, phenformin, etomoxir, combination injection, siRNA, shRNA

C. DOCUMENTS CONSIDERED TO BE RELEVANT

| Category* | Citation of document, with indication, where appropriate, of the relevant passages | Relevant to claim No. |
|---|---|---|
| X | LEE, Jae-Seon et al., "Dual Targeting of Glutaminase 1 and Thymidylate Synthase Elicits Death Synergistically in NSCLC", Cell Death & Disease, 2016, vol. 7, no. 12, e2511, inner pages 1-13<br>See inner page 3; and figures 2f, 3b, 3c, 6c. | 1-11,14,15 |
| X | US 2015-0157622 A1 (BASILEA PHARMACEUTICA AG. et al.) 11 June 2015<br>See paragraphs [0084], [0095], [0096]. | 1,2,6-11,14,15 |
| A | KANG, Joon Hee et al., "Aldehyde Dehydrogenase Inhibition Combined with Phenformin Treatment Reversed NSCLC through ATP Depletion", Oncotarget, 2016, vol. 7, no. 31, pages 49397-49410<br>See the entire document. | 1-11,14,15 |
| A | US 9539323 B2 (SUKHATME, Vikas P. et al.) 10 January 2017<br>See the entire document. | 1-11,14,15 |
| A | LYTOVCHENKO, Oleksandr et al., "Expression and Putative Role of Mitochondrial Transport Proteins in Cancer", Biochimica et Biophysica Acta (BBA)-Bioenergetics, 22 March 2017, vol. 1858, no. 8, pages 641-654<br>See the entire document. | 1-11,14,15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 JULY 2018 (16.07.2018) | **16 JULY 2018 (16.07.2018)** |
| Name and mailing address of the ISA/KR<br>Korean Intellectual Property Office<br>Government Complex-Daejeon, 189 Seonsa-ro, Daejeon 302-701, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**

International application No.
**PCT/KR2018/004344**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **12, 13**
because they relate to subject matter not required to be searched by this Authority, namely:
Claims 12 and 13 pertain to a method for treatment of the human body by therapy, and thus pertain to subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT
Information on patent family members

International application No.
PCT/KR2018/004344

| Patent document cited in search report | Publication date | Patent family member | Publication date |
|---|---|---|---|
| US 2015-0157622 A1 | 11/06/2015 | AU 2013-289384 A1 | 22/01/2015 |
| | | AU 2013-289384 B2 | 14/04/2016 |
| | | BR 112015000179 A2 | 27/06/2017 |
| | | CA 2878605 A1 | 16/01/2014 |
| | | CN 104603133 A | 06/05/2015 |
| | | CN 104603133 B | 02/11/2016 |
| | | EP 2872505 A1 | 20/05/2015 |
| | | IL 236321 A | 26/02/2015 |
| | | JP 2015-522048 A | 03/08/2015 |
| | | KR 10-2015-0041786 A | 17/04/2015 |
| | | MX 2015000309 A | 14/08/2015 |
| | | RU 2015104122 A | 27/08/2016 |
| | | US 9511064 B2 | 06/12/2016 |
| | | WO 2014-009222 A1 | 16/01/2014 |
| US 9539323 B2 | 10/01/2017 | US 2013-0209488 A1 | 15/08/2013 |
| | | WO 2012-019154 A2 | 09/02/2012 |
| | | WO 2012-019154 A3 | 20/03/2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020170048558
- KR 101579371
- KR 10145806


### Non-patent literature cited in the description


- **GREENHOUSE, WALTER VV ; ALBERT L. LEHNINGER.** *Cancer Research,* 1976, vol. 36.4, 1392-1396
- **NAIK ; RAVI et al.** *Journal of medicinal chemistry,* 2017, vol. 60.20, 8631-8646
- **ELEFTHERIADIS ; THEODOROS et al.** *Experimental and therapeutic medicine,* 2015, vol. 10.5, 1959-1966
- **GUTMAN, MENACHEM ; ESTER HARTSTEIN.** *FEBS letters,* 1974, vol. 49.2, 170-173
- **SCHINDLER, PETER W.** *The FEBS Journal,* 1975, vol. 51.2, 579-585
- **WANG ; CAIXIA et al.** *Cancer letters,* 2016, vol. 378.1, 1-7
- **YAMADA ; RYO-HEI et al.** *Biochimica et BiophysicaActa (BBA)-General Subjects,* 1984, vol. 801.1, 151-154
- **RANDO, ROBERT R.** *Biochemistry,* 1974, vol. 13.19, 3859-3863
- **CAPOBIANCO ; LOREDANA et al.** *Biochemistry,* 1996, vol. 35.27, 8974-8980

```
<110>   NATIONAL CANCER CENTER

<120>   pharmaceutical composition for prevention or treatment of cancer
        comprising malate-aspartate shuttle inhibitor and chemotherapy

<130>   1063784

<150>   KR 10-2017-0048558
<151>   2017-04-14

<160>   6

<170>   KopatentIn 2.0

<210>   1
<211>   27
<212>   RNA
<213>   Artificial Sequence

<220>
<223>   SLC25A11_1_sense


<400>   1
ggaauacaag aacgggcugg acguguu                                          27


<210>   2
<211>   27
<212>   RNA
<213>   Artificial Sequence

<220>
<223>   SLC25A11_1_antisense


<400>   2
cacguccagc ccguucuugu auuccuu                                          27


<210>   3
<211>   27
<212>   RNA
<213>   Artificial Sequence

<220>
<223>   SLC25A11_2_sense


<400>   3
acauugccaa gacccgaauc cagaauu                                          27


<210>   4
<211>   27
<212>   RNA
<213>   Artificial Sequence

<220>
<223>   SLC25A11_2_antisense


<400>   4
```

uucuggauuc gggucuuggc aauguuu 27

<210> 5
<211> 58
<212> RNA
<213> Artificial Sequence

<220>
<223> TRCN0000322801

<400> 5
ccggtacccg ccttggcatc tatacctcga ggtatagatg ccaaggcggg tatttttg 58

<210> 6
<211> 58
<212> RNA
<213> Artificial Sequence

<220>
<223> TRCN0000044416

<400> 6
ccgggcagat gaacaaggcc tacaactcga gttgtaggcc ttgttcatct gctttttg 58

SEQUENCE LISTING

<110> NATIONAL CANCER CENTER

<120> PHARMACEUTICAL COMPOSITION FOR PREVENTING AND TREATING CANCER, CONTAINING MALATE-ASPARTATE SHUTTLE INHIBITOR AND ANTICANCER DRUG AS ACTIVE INGREDIENTS

<130> SOP115021EP

<150> PCT/KR2018/004344
<151> 2018-04-13

<160> 6

<170> PatentIn version 3.2

<210> 1
<211> 27
<212> RNA
<213> Artificial

<220>
<223> SLC25A11_1_sense

<400> 1
ggaauacaag aacgggcugg acguguu 27

<210> 2
<211> 27
<212> RNA
<213> Artificial

<220>
<223> SLC25A11_1_antisense

<400> 2
cacguccagc ccguucuugu auuccuu 27

<210> 3
<211> 27
<212> RNA
<213> Artificial

<220>
<223> SLC25A11_2_sense

<400> 3
acauugccaa gacccgaauc cagaauu 27

<210> 4
<211> 27
<212> RNA
<213> Artificial

<220>
<223> SLC25A11_2_antisense

<400> 4
uucuggauuc gggucuuggc aauguuu 27

```
<210>  5
<211>  58
<212>  DNA
<213>  Artificial

<220>
<223>  TRCN0000322801

<400>  5
ccggtacccg ccttggcatc tatacctcga ggtatagatg ccaaggcggg tatttttg       58


<210>  6
<211>  58
<212>  DNA
<213>  Artificial

<220>
<223>  TRCN0000044416

<400>  6
ccgggcagat gaacaaggcc tacaactcga gttgtaggcc ttgttcatct gctttttg       58
```